# EUROPEAN PATENT SPECIFICATION

(11) **EP 2 872 184 B1**
(45) Date of publication and mention of the grant of the patent: **16.09.2020**
(21) Application number: 13816739.0
(22) Date of filing: 12.07.2013
(51) Int. Cl.: A61K 48/00, A61P 37/06, A61K 35/17, A61K 35/12, A61K 38/17

(54) **CART19 FOR USE IN A METHOD TO DEPLETE NORMAL B CELLS TO INDUCE TOLERANCE**
CART19 ZUR VERWENDUNG ZUR DEZIMIERUNG NORMALER B-ZELLEN ZUR INDUKTION EINER TOLERANZ
CART19 POUR UTILISATION DE DÉPLÉTER DES LYMPHOCYTES B NORMAUX POUR INDUIRE LA TOLÉRANCE

(30) Priority: 13.07.2012 US 201261671508 P
(43) Date of publication of application: 20.05.2015
(73) Proprietor: The Trustees of The University of Pennsylvania, Philadelphia PA 19104-6283 (US)
(72) Inventor: LEVINE, Bruce, L., Cherry Hill, NJ 08003 (US); KALOS, Michael, D., New York, NY 10022 (US); JUNE, Carl, H., Merion Station, PA 19066 (US)
(74) Representative: Bassil, Nicholas Charles
(86) International application number: PCT/US2013/050293
(87) International publication number: WO 2014/012001

(56) References cited:
- WO-A1-2011/097477
- WO-A1-2012/079000
- WO-A2-2011/059836
- WO-A2-2012/082841
- US-A1- 2009 148 419
- DAVID L PORTER ET AL: "Chimeric Antigen Receptor Therapy for B-cell Malignancies", JOURNAL OF CANCER, vol. 2042127, 1 January 2011 (2011-01-01), page 331, XP055247868, AU ISSN: 1837-9664, DOI: 10.7150/jca.2.331
- David L. Porter ET AL: "Chimeric Antigen Receptor-Modified T Cells in Chronic Lymphoid Leukemia", New England Journal of Medicine, vol. 365, no. 8, 25 August 2011 (2011-08-25), pages 725-733, XP055052475, ISSN: 0028-4793, DOI: 10.1056/NEJMoa1103849

## Description

### BACKGROUND OF THE INVENTION

Using gene transfer technologies, T cells can be genetically modified to stably express antibody binding domains on their surface that confer novel antigen specificities that are major histocompatibility complex (MHC)-independent. Chimeric antigen receptors (CARs) are an application of this approach that combines an antigen recognition domain of a specific antibody with an intracellular domain of the CD3-z chain or FcgRI protein into a single chimeric protein (Gross et al., 1989 Proc. Natl. Acad. Sci. U.S.A. 86: 10024-10028; Irving et al., 1991 Cell 64: 891-901). Trials testing CARs are presently under way at a number of academic medical centers (Kohn et al. 2011 Mol. Ther. 19: 432-438; Jena et al., 2010 Blood 116: 1035-1044). In most cancers, tumor-specific antigens are not yet well defined, but in B cell malignancies, CD 19 is an attractive tumor target. Expression of CD 19 is restricted to normal and malignant B cells (Uckun et al., 1988 Blood 71: 13-29), and CD19 is a widely accepted target to safely test CARs. Although CARs can trigger T cell activation in a manner similar to an endogenous T cell receptor, a major impediment to the clinical application of this technology to date has been the limited in vivo expansion of CAR+ T cells, rapid disappearance of the cells after infusion, and disappointing clinical activity (Jena et al., 2010 Blood 116: 1035-1044; Sadelain et al., 2009 Curr. Opin. Immunol. 21: 215-223).

CAR-mediated T cell responses may be further enhanced with addition of costimulatory domains. In a preclinical model, inclusion of the CD137 (4-1BB) signaling domain was found to significantly increased antitumor activity and in vivo persistence of CARs compared to inclusion of the CD3-z chain alone (Milone et al., 2009 Mol. Ther. 17, 1453-1464; Carpenito et al., 2009 Proc. Natl. Acad. Sci. U.S.A. 106: 3360-3365). To evaluate the safety and feasibility for adoptive transfer of T cells gene-modified to express such CARs, a pilot clinical trial using autologous T cells expressing an anti-CD 19 CAR including both CD3-z and the 4-1BB costimulatory domain (CART 19 cells) to target CD 19+ malignancies was conducted. Three patients have been treated under this protocol. Some of the findings from one of these patients are described in (Porter et al., 2011 N. Engl. J. Med. 365: 8), which reports that this treatment results in tumor regression, CART19 cell persistence, and the unexpected occurrence of delayed tumor lysis syndrome. It was also observed that the CART19 cells mediated potent clinical antitumor effects in all three patients treated. On average, each infused CAR T cell and/or their progeny eliminated more than 1000 leukemia cells in vivo in patients with advanced chemotherapy-resistant chronic lymphocytic leukemia (CLL). CART19 cells underwent robust in vivo T cell expansion, persisted at high levels for at least 6 months in blood and bone marrow (BM), continued to express functional receptors on cells with a memory phenotype, and maintained anti-CD 19 effector function in vivo. However, it still remains unclear how the CART19 cells evade the rejection by the human host given that the CAR19 construct contains both murine sequences (the antibody determinants) and unique junctional fragments between the different components of the CAR19 construct.

Thus, there still remains a need in the art as to the mechanism of long term persistence of the CART19 cells and why these cells are not rejected by the human host. The present invention addresses this need.

### SUMMARY OF THE INVENTION

According to a first aspect of the invention, there is provided a cell genetically modified to express an anti-CD19 CAR wherein the CAR comprises a CD19 antigen binding domain, a costimulatory signaling region, and a CD3 zeta signaling domain, for use in a method for preventing or delaying rejection of a transplanted tissue or organ in a subject, the method comprising administering to the subject an effective amount of said genetically modified cell before, at the same time as, or after the administration of the transplanted tissue or organ, wherein the CD19 antigen binding domain targets the CD19 B cell surface marker, wherein the genetically modified cell depletes B cells, and wherein B cell aplasia is sustained for eighteen months after the administration of the genetically modified cell to the subject, wherein the tissue or organ is heart, heart valve, lung, kidney, liver, pancreas, intestine, skin, blood vessel, bone marrow, stem cell, bone or islet cell.

In accordance with the first aspect of the invention, the method for preventing or delaying rejection of a transplanted tissue or organ in a subject may further comprise administration of a genetically modified cell to said tissue or organ before said tissue or organ is transplanted into the subject. The method for preventing or delaying rejection of a transplanted tissue or organ in a subject may further comprise evaluation of the presence of CAR expressing cells in the subject at least once at twelve or eighteen months after the administration of the cell genetically modified to express an anti CD19 CAR. The method for preventing or delaying rejection of a transplanted tissue or organ in a subject may further comprise the evaluation of the depletion of B cells in the subject at least once at twelve or eighteen months after the administration of the cell genetically modified to express an anti-CD19 CAR.

According to a second aspect of the invention, there is provided a cell genetically modified to express an anti-CD19 CAR, wherein the CAR comprises a CD19 antigen binding domain, a costimulatory signaling region, and a CD3 zeta signaling domain, for use in a method for treating graft versus host disease (GVHD) in a subject having received a tissue or organ transplant, the method comprising administering to the subject an effective amount of said genetically modified cell before, at the same time as, or after the administration of the transplanted tissue or organ, wherein the antigen binding domain targets a CD19 B cell surface marker, wherein the genetically modified cell depletes B cells, thereby treating GVHD in the subject, and wherein B cell aplasia is sustained for eighteen months after the administration of the genetically modified cell to the subject, wherein the tissue or organ is heart, heart valve, lung, kidney, liver, pancreas, intestine, skin, blood vessel, bone marrow, stem cell, bone or islet cell.

In accordance with the second aspect of the invention, the genetically modified cell for use the genetically modified cell may be is administered at the same time as a transplanted tissue or organ, before the administration of the transplanted tissue or organ, or after the administration of the transplanted tissue or organ. The method for treating graft versus host disease (GVHD) may further comprise administration of said genetically modified cell to a tissue or organ before said tissue or organ is transplanted into the subject. The method for treating graft versus host disease (GVHD) may further comprise the evaluation of the presence of CAR expressing cells in the subject at least once at twelve or eighteen months after the administration of the cell genetically modified to express an anti-CD19 CAR. The method for treating graft versus host disease (GVHD) may further comprise the evaluation of the depletion of B cells in the subject at least once at twelve or eighteen months after the administration of the cell genetically modified to express an anti-CD19 CAR.

The disclosure describes a method of depleting B cells in a subject. In one embodiment, the method comprises administering to a subject an effective amount of a cell genetically modified to express a CAR wherein the CAR comprises an antigen binding domain, a costimulatory signaling region, and a CD3 zeta signaling domain, wherein the antigen binding domain targets a B cell surface marker, thereby depleting B cells in the subject.

The disclosure describes a method of promoting tolerance in a subject. In one embodiment, the method comprises administering to a subject an effective amount of a cell genetically modified to express a CAR wherein the CAR comprises an antigen binding domain, a costimulatory signaling region, and a CD3 zeta signaling domain, wherein the antigen binding domain targets a B cell surface marker, thereby promoting tolerance in the subject.

In one embodiment, the tolerance is transplant tolerance to a transplanted tissue.

In one embodiment, the genetically modified cell depletes B cells.

In one embodiment, the genetically modified cell is administered at the same time as the transplanted tissue.

In one embodiment, the genetically modified cell is administered before the administration of the transplanted tissue.

In one embodiment, the genetically modified cell is administered after the administration of transplanted tissue.

The disclosure describes a method for treating graft versus host disease (GVHD). In one embodiment, the method comprises administering a cell genetically modified to express a CAR to a subject in need thereof, wherein the CAR comprises an antigen binding domain, a costimulatory signaling region, and a CD3 zeta signaling domain, wherein the antigen binding domain targets a B cell surface marker, thereby treating GVHD in the subject.

In one embodiment, the genetically modified cell depletes B cells.

In one embodiment, the genetically modified cell is administered at the same time as a transplanted tissue.

In one embodiment, the genetically modified cell is administered before the administration of the transplanted tissue.

In one embodiment, the genetically modified cell is administered after the administration of the transplanted tissue.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.

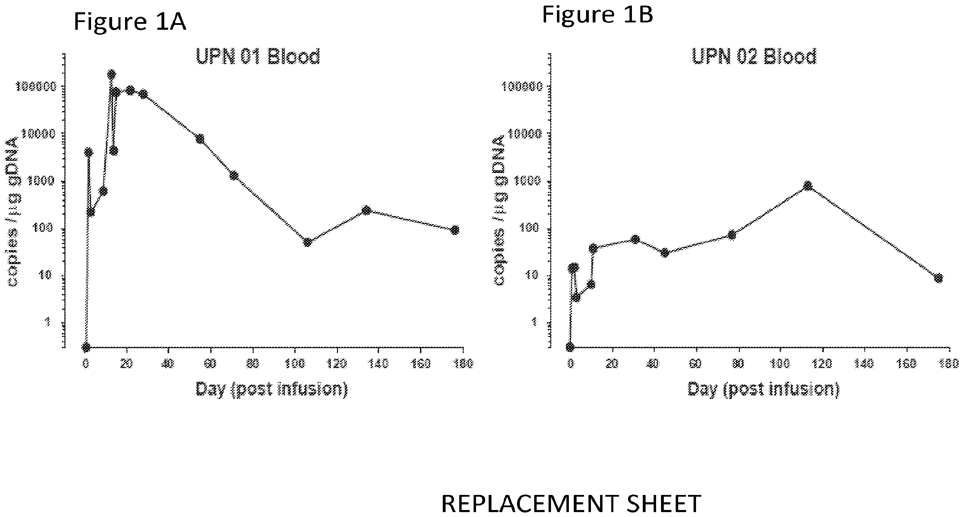

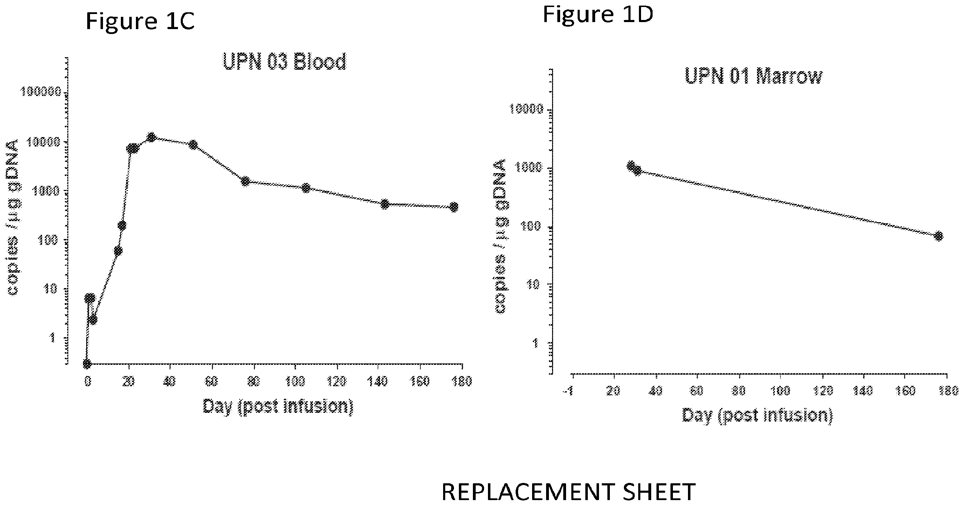

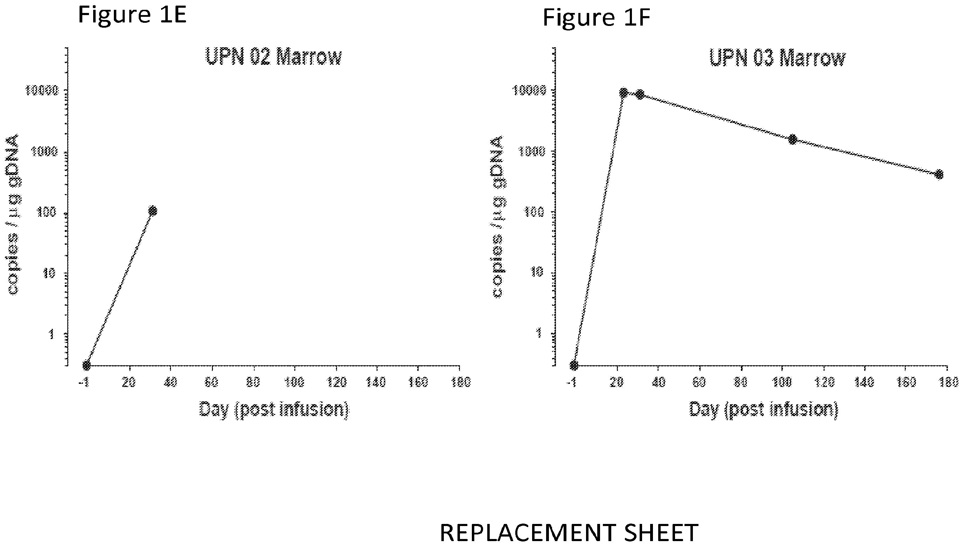

Figure 1, comprising Figures 1A through 1F, is a series of images demonstrating sustained *in vivo* expansion and persistence in blood and marrow of CART19 cells. DNA isolated from whole blood as depicted in Figure 1A through 1C or marrow as depicted in Figure ID through IF, samples obtained from UPN 01 as depicted in Figure 1A and 1D, UPN 02 as depicted in Figure 1B and 1E and UPN 03 as depicted in Figure 1C and IF was subjected in bulk to Q-PCR analysis using a qualified assay to detect and quantify CART19 sequences. Each data point represents the average of triplicate measurements on 100-200 ng genomic DNA, with maximal % CV less than 1.56%. Pass/fail parameters for the assay included pre-established ranges for slope and efficiency of amplification, and amplification of a reference sample. The lower limit of quantification for the assay established by the standard curve range was 2 copies transgene/microgram genomic DNA; sample values below that number are considered estimates and presented if at least 2/3 replicates generated a Ct value with % CV for the values 15%. CART19 cells were infused at day 0, 1, and 2 for UPN 01 and UPN 03, and days 0, 1, 2 and 11 for UPN 02.

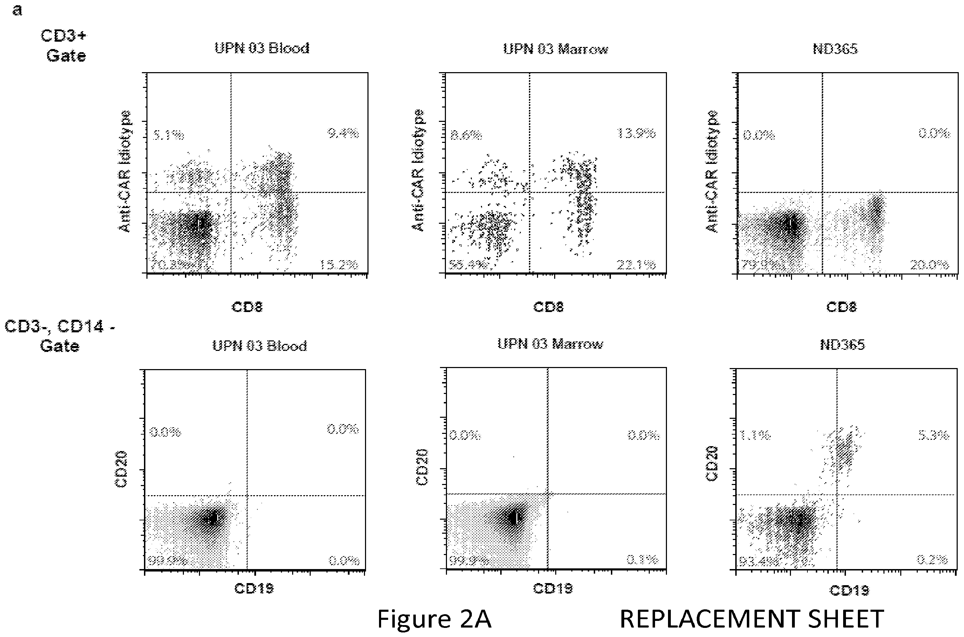

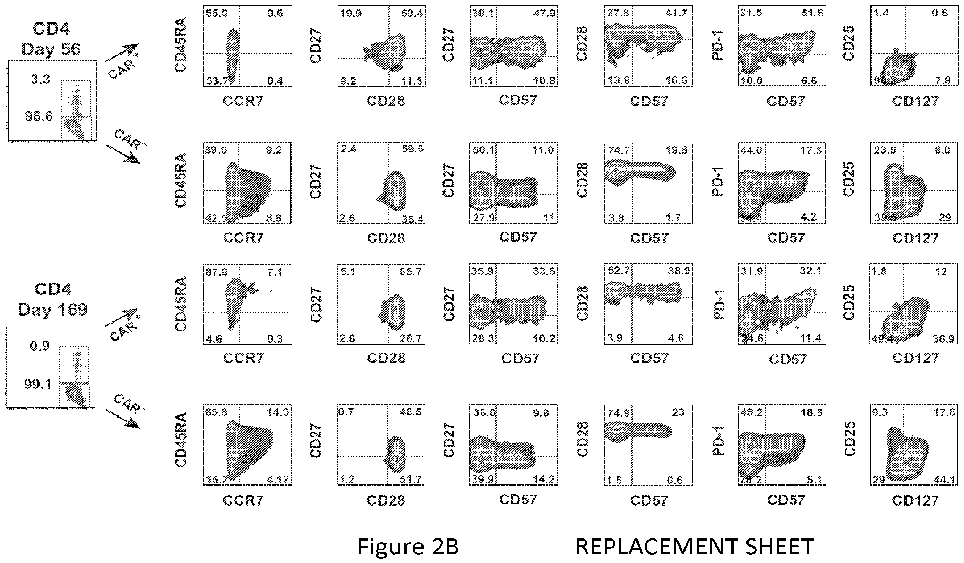

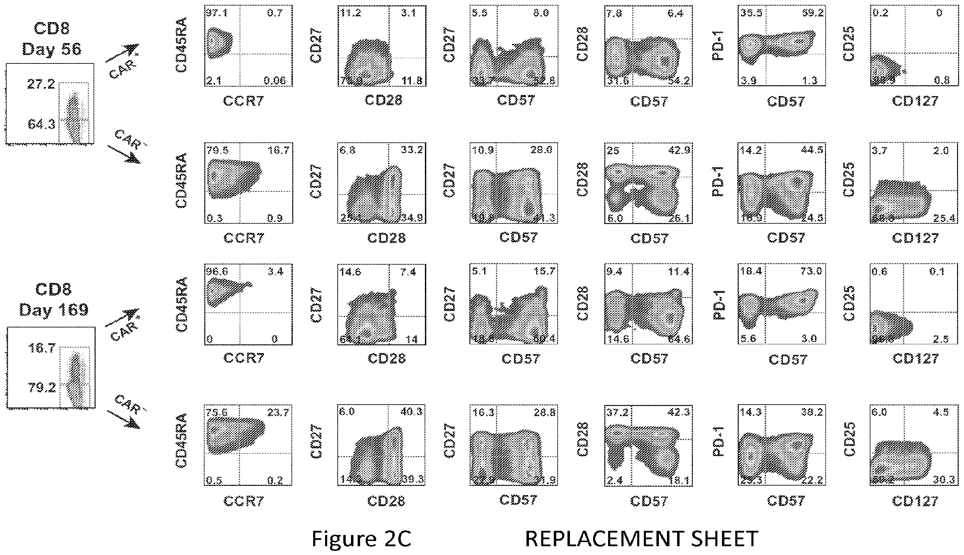

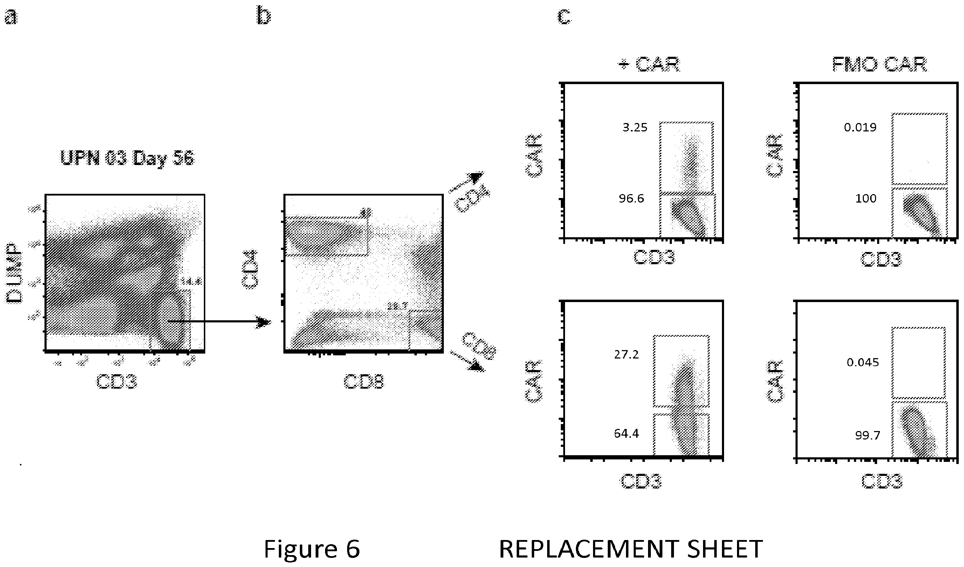

Figure 2, comprising Figures 2A through 2C, is a series of images depicting prolonged surface CART19 expression and establishment of functional memory CARs *in vivo.* Figure 2A depicts detection of CAR-expressing CD3+ lymphocytes and absence of B cells in periphery and marrow. Freshly processed peripheral blood or marrow mononuclear cells obtained from UPN 03 at day 169 post-CART19 cell infusion were evaluated by flow-cytometry for surface expression of CAR19 (top) or presence of B cells (bottom); as a control, PBMC obtained from a healthy donor ND365 were stained. To evaluate CAR19 expression in CD3+ lymphocytes, samples were co-stained with antibodies to CD14-PE-Cy7 and CD16-PE-Cy7 (dump channel) and CD3-FITC, positively gated on CD3+, and evaluated for CAR19 expression in the CD8+ and CD8-lymphocyte compartments by co-staining with CD8a-PE and the anti-CAR19 idiotype antibody conjugated to Alexa-647. Data in plots are gated on the dump channel-negative/CD3-positive cell population. To evaluate the presence of B cells, samples were co-stained with antibodies to CD14-APC and CD3-FITC (dump channels) and evaluated for the presence of B cells in the dump channel-negative fraction by co-staining with antibodies to CD20-PE and CD19-PE-Cy-7. In all cases, negative gate quadrants were established on no-stain controls as depicted in Figures 2B and 2C. T cell immunophenotyping of CD4+ (Figure 2B) and CD8+ (Figure 2C) T cell subsets is shown. Frozen peripheral blood samples from UPN 03 obtained by apheresis at day 56 and 169 post T cell infusion were rested overnight in culture medium with no added factors, washed, and subjected to multi-parametric immunophenotyping for expression of markers of T cell memory, activation, and exhaustion. The gating strategy, as depicted in Figure 6, involved an initial gating on dump channel (CD 14, CD 16, Live/Dead Aqua)-negative and CD3-positive cells, followed by positive gates on CD4+ and CD8+ cells. Gates and quadrants were established using FMO controls (CAR, CD45RA, PD-1, CD25, CD127, CCR7) or by gating on positive cell populations (CD3, CD4, CD8) and clearly delineated subsets (CD27, CD28, CD57); data were displayed after bi-exponential transformation for objective visualization of events. Functional competence of persisting CAR cells were shown in the following experiments. Frozen peripheral blood samples from UPN 03 obtained by apheresis at day 56 and 169 post T cell infusion were rested overnight in culture medium with no added factors, washed, and evaluated directly *ex-vivo* for the ability to recognize CD19-expressing target cells using CD107 degranulation assays. Following a two-hour incubation in the presence of anti-CD28, anti-CD49d, and CD107-FITC, cell mixtures were harvested, washed, and subjected to multi-parametric flow cytometric analysis to evaluate the ability of CART19 cells to de-granulate in response to CD19-expressing targets. The gating strategy involved an initial gate on dump channels (CD14-PE-Cy7, CD16-PE-Cy7, Live/Dead Aqua)-negative and CD3-PE-positive cells, followed by gating on CD8-PE-Texas Red-positive cells; presented data is for the CD8+ gated population. In all cases, negative gate quadrants were established on no-stain controls.

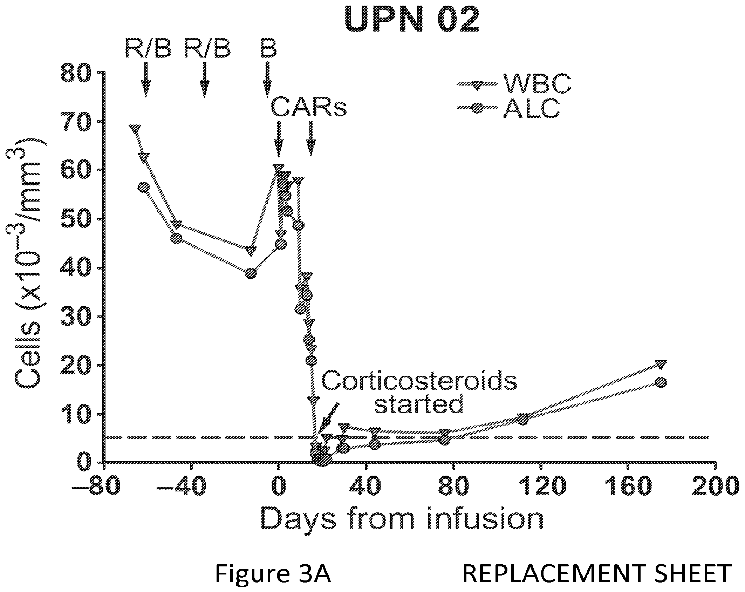

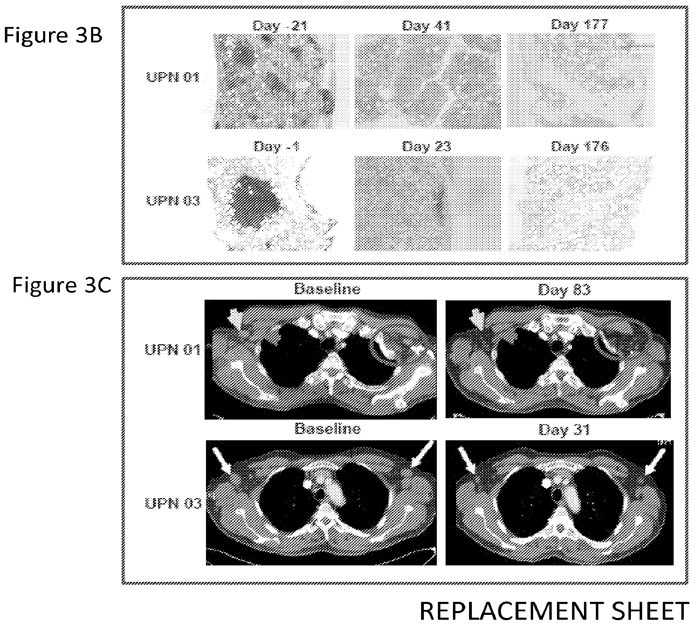

Figure 3, comprising Figures 3A through 3C, is series of images depicting the results of experiments evaluating clinical responses after infusion of CART19 cells. Figure 3A depicts that UPN 02 was treated with two cycles of rituximab and bendamustine with minimal response (R/B, arrow). CART19 T cells were infused beginning 4 days after bendamustine only (B, arrow). The rituximab and bendamustine-resistant leukemia was rapidly cleared from blood, as indicated by a decrease in the absolute lymphocyte count (ALC) from 60,600/µl to 200/µl within 18 days of the infusion. Corticosteroid treatment was started on day 18 post infusion due to malaise and non-infectious febrile syndrome. The reference line (dotted) indicates upper limit of normal for ALC. Figure 3B depicts the results of example experiments staining sequential bone marrow biopsy or clot specimens from patient UPN 01 and 03 for CD20. Pretreatment infiltration with leukemia present in both patients was absent on post treatment specimens accompanied by normalization of cellularity and tri-lineage hematopoiesis. UPN 01 has not had any CLL cells detected as assessed by flow cytometry, cytogenetics and fluorescence in-situ hybridization or normal B cells detected by flow cytometry in bone marrow or blood. UPN 03 had 5% residual normal CD5-negative B cells confirmed by flow cytometry on day +23, which also showed them to be polyclonal; no normal B cells were detected at day +176. Figure 3C depicts the results of experiments using sequential CT imaging to assess the rapid resolution of chemotherapy-resistant generalized lymphadenopathy. Bilateral axillary masses resolved by 83 (UPN 01) and 31 (UPN 03) days post infusion, as indicated by arrows and circle.

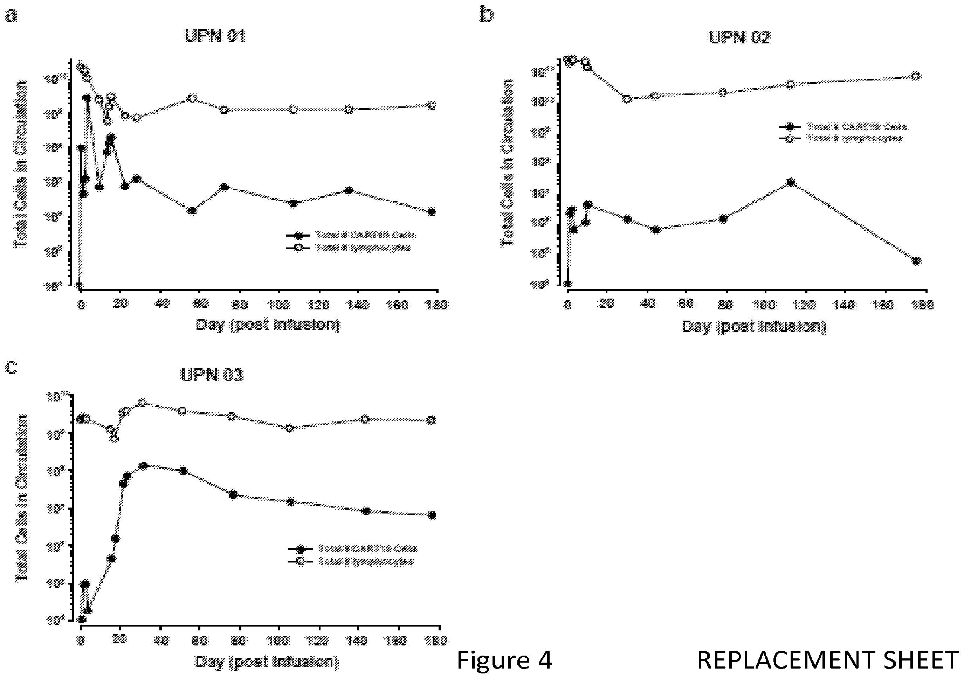

Figure 4, comprising Figures 4A through 4C, is a series of images depicting absolute lymphocyte counts and total CART19+ cells in circulation for UPN 01, 02, 03. The total number of lymphocytes (Total normal and CLL cells) vs. Total CART19+ cells in circulation is plotted for all 3 subjects using the absolute lymphocyte count from CBC values, and assuming a 5.0 L volume of blood. The total number of CART19 cells in circulation was calculated by using the tandem CBC values with absolute lymphocyte counts and the Q-PCR marking values as depicted in Figure 1, converting copies/µg DNA to average % marking as described elsewhere herein. The Q-PCR % marking was found to correlate closely (<2 fold variation) with the flow cytometric characterization of the infusion products and with data from samples where concomitant flow cytometry data was available to directly enumerate CART19 cells by staining.

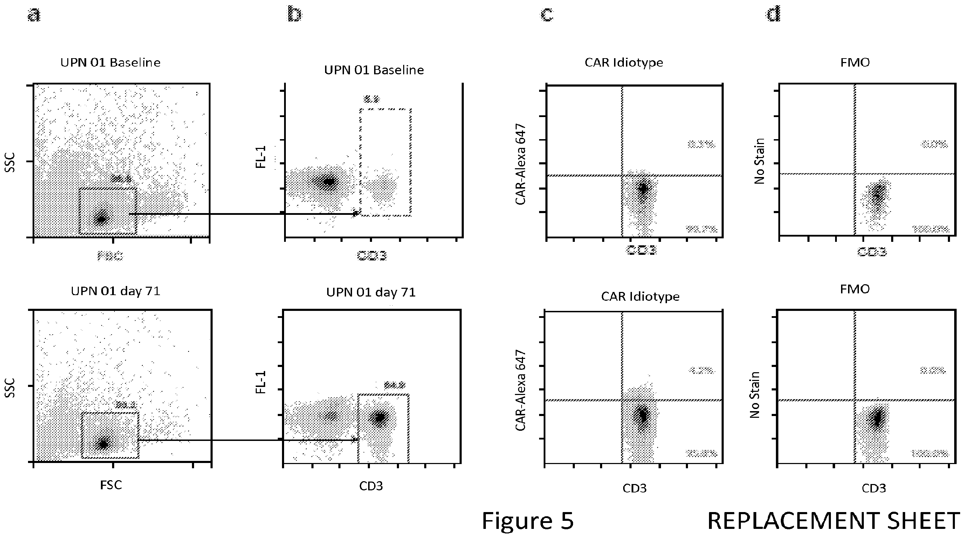

Figure 5, comprising Figures 5A through 5D is a series of images depicting experiments involving the direct *ex vivo* detection of CART19-positive cells in UPN-01 PBMC 71 days post-T cell infusion. UPN-01 PBMC collected either fresh post-apheresis on day71 day post infusion, or frozen at the time of apheresis for manufacture of the T cell product(baseline) and viably thawed prior to the staining, were subjected to flow-cytometric analysis to detect the presence of CART19 cells that express the CAR19 moiety on the surface. To evaluate the expression of CAR19 in lymphocytes, samples were co-stained with CD3-PE and the anti-CAR19 idiotype antibody conjugated to Alexa-647, or co-stained with CD3-PE alone (FMO for CAR19). Figure 5A depicts that an initial lymphocyte gate was established based on forward and side scatter (FSC vs. SSC), followed by gating on CD3+ cells. Figure 5B depicts CD3+ lymphocyte gate; Figure 5C depicts CAR idiotype stain; Figure 5D depicts CAR idiotype FMO. The CAR19-positive gate was established on the CAR19 FMO samples.
Figure 6, comprising Figures 6A through 6C, is a series of images depicting the gating strategy to identify CART19 expression by using polychromatic flow cytometry in UPN 03 blood specimens. The gating strategy for Figure 6C is shown for the UPN 03 Day 56 sample and is representative of the strategy used on the UPN 03 Day 169 sample. Figure 6A depicts primary gate: Dump (CD14, CD16, LIVE/dead Aqua) negative, CD3-positive. Figure 6B depicts secondary gates: CD4-positive, CD8-positive. Figure 6C depicts tertiary gates: CAR19-positive and CAR19-negative, established on CAR FMO samples (right-most panels).

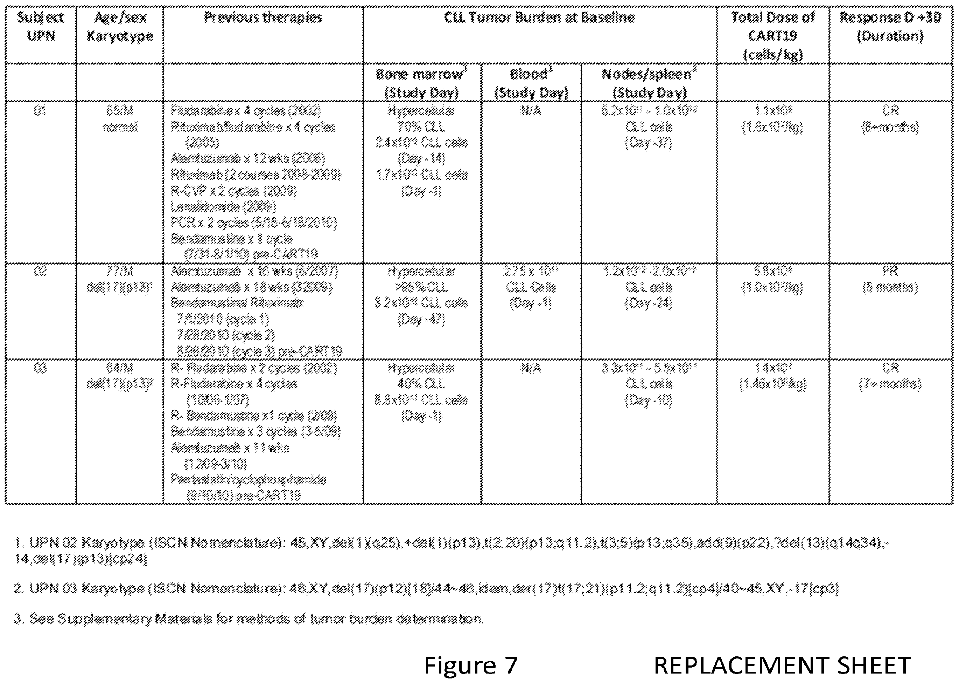

Figure 7 is an image summarizing the patient demographics and response.

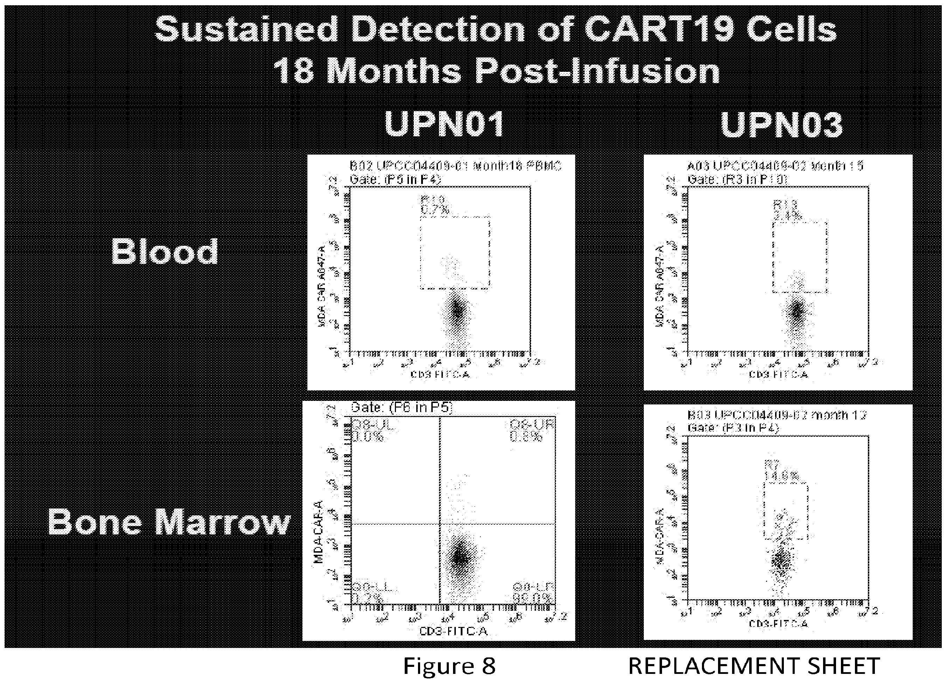

Figure 8 is an image depicting long term expression of CART19.

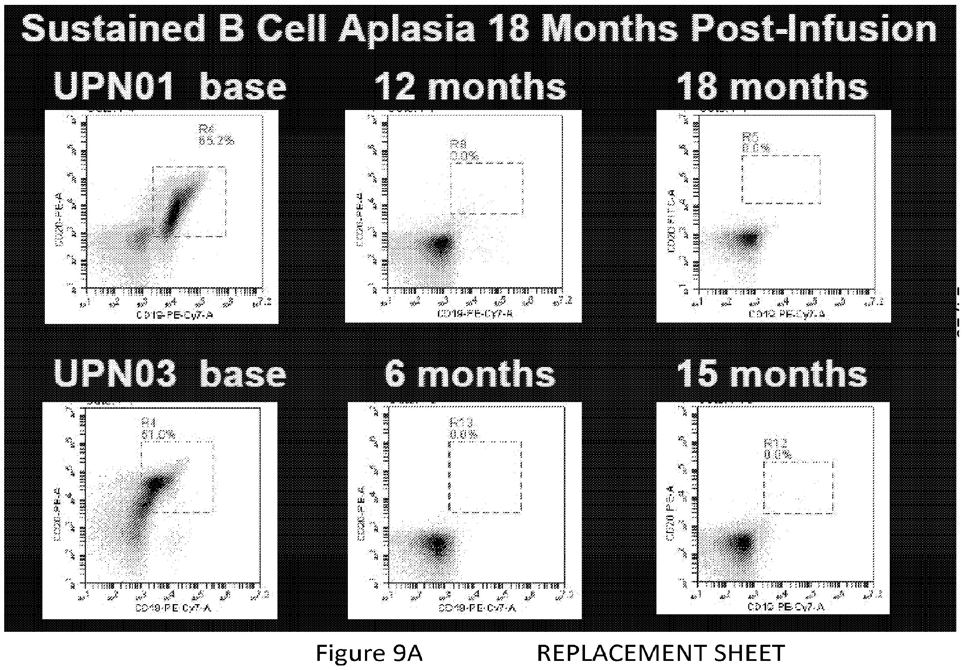

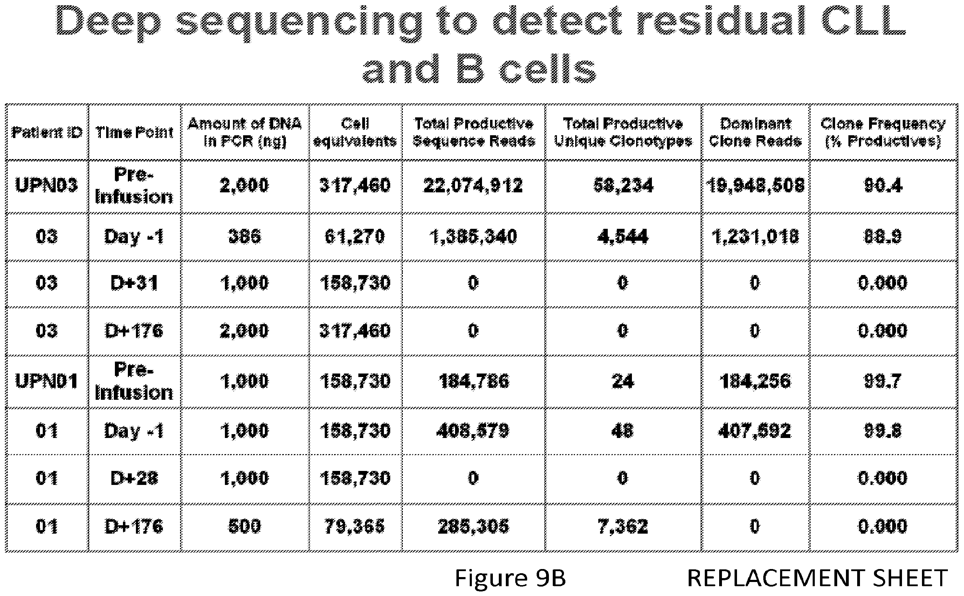

Figure 9, comprising Figures 9A and 9B, is a series of images depicting deep B cell aplasia.

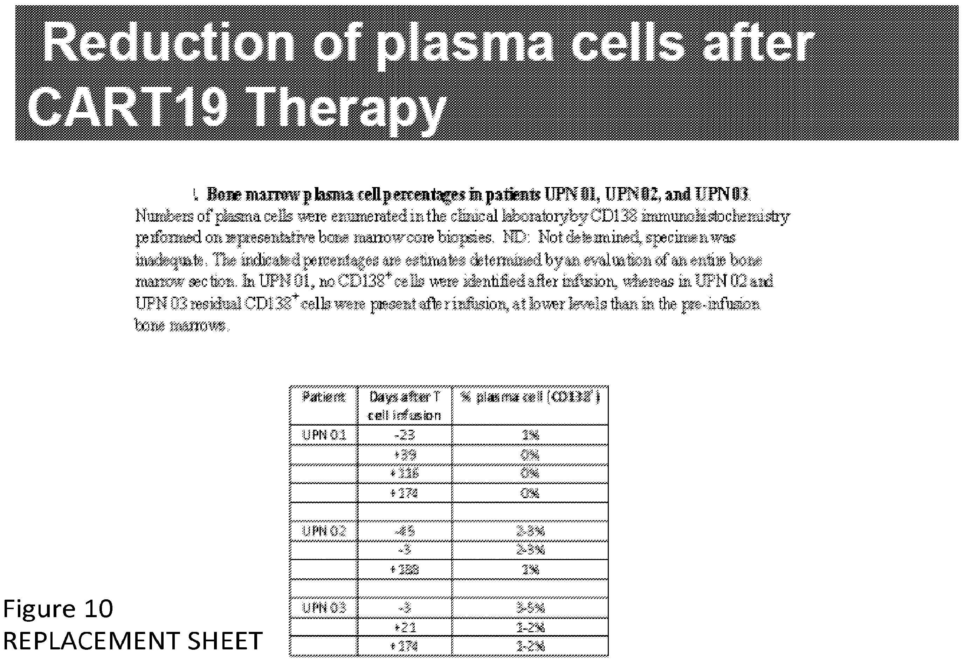

Figure 10 is an image demonstrating a reduction in plasma cells in all 3 patients.

### DETAILED DESCRIPTION

The present invention is based in part on the surprising discovery that T cells expressing an anti-CD19 CAR including both CD3z and the 4-1BB costimulatory domain (CART 19 cells) persisted in a mammalian host for a long period time. For example, at this time, cells expressing surface CAR19 have been observed to be present in a mammalian host for over 21 months after CAR19 T cell infusion. Accordingly, the present disclosure describes a method for depleting normal B cells in a mammal by administering to the mammal in need thereof a CAR that targets B cells in order to induce tolerance in the mammal.

The disclosure relates to compositions and methods for depleting B cells, and therefore inducing tolerance. The present disclosure relates to a method of adoptive cell transfer of T cells transduced to express a chimeric antigen receptor (CAR). CARs are molecules that combine antibody-based specificity for a target antigen (e.g., B cell antigen) with a T cell receptor-activating intracellular domain to generate a chimeric protein that exhibits a specific anti-B cell cellular immune activity.

In one embodiment, the CAR described herein comprises an extracellular domain having an antigen recognition domain that targets a B cell antigen, a transmembrane domain, and a cytoplasmic domain.

In one embodiment, the CAR T cells described herein can be generated by introducing a lentiviral vector comprising a desired CAR. The CAR T cells described herein are able to replicate *in vivo* resulting in long-term persistence that can lead to sustained B cell depletion and tolerance.

In one embodiment the disclosure relates to administering a genetically modified T cell expressing a CAR to effectively reduce the incidence, severity, or duration of graft versus host disease (GVHD), a rejection episode, or post-transplant lymphoproliferative disorder.

### Definitions

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

It is also to be understood that the terminology used herein is for the purpose of describing particular embodiments only, and is not intended to be limiting.
The articles "a" and "an" are used herein to refer to one or to more than one (*i.e.,* to at least one) of the grammatical object of the article. By way of example, "an element" means one element or more than one element.
"About" as used herein when referring to a measurable value such as an amount, a temporal duration, and the like, is meant to encompass variations of ±20% or ±10%, in some instances ±5%, in some instances ±1%, and in some instances ±0.1% from the specified value, as such variations are appropriate to perform the disclosed methods.
"Activation," as used herein, refers to the state of a T cell that has been sufficiently stimulated to induce detectable cellular proliferation. Activation can also be associated with induced cytokine production, and detectable effector functions. The term "activated T cells" refers to, among other things, T cells that are undergoing cell division.
The term "antibody," as used herein, refers to an immunoglobulin molecule which specifically binds with an antigen. Antibodies can be intact immunoglobulins derived from natural sources or from recombinant sources and can be immunoreactive portions of intact immunoglobulins. Antibodies are often tetramers of immunoglobulin molecules. The antibodies in the present invention may exist in a variety of forms including, for example, polyclonal antibodies, monoclonal antibodies, Fv, Fab and F(ab)₂, as well as single chain antibodies and humanized antibodies (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426).
The term "antibody fragment" refers to a portion of an intact antibody and refers to the antigenic determining variable regions of an intact antibody. Examples of antibody fragments include, but are not limited to, Fab, Fab', F(ab')2, and Fv fragments, linear antibodies, scFv antibodies, and multispecific antibodies formed from antibody fragments.
The term "antigen" or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to elicit the desired immune response. Moreover, a skilled artisan will understand that an antigen need not be encoded by a "gene" at all. It is readily apparent that an antigen can be generated synthesized or can be derived from a biological sample. Such a biological sample can include, but is not limited to a tissue sample, a tumor sample, a cell or a biological fluid.
The term "auto-antigen" means, in accordance with the present invention, any self-antigen which is recognized by the immune system as if it were foreign. Auto-antigens comprise, but are not limited to, cellular proteins, phosphoproteins, cellular surface proteins, cellular lipids, nucleic acids, glycoproteins, including cell surface receptors.
The term "autoimmune disease" as used herein is defined as a disorder that results from an autoimmune response. An autoimmune disease is the result of an inappropriate and excessive response to a self-antigen. Examples of autoimmune diseases include but are not limited to, Addision's disease, alopecia greata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, among others.
As used herein, the term "autologous" is meant to refer to any material derived from the same individual to which it is later to be re-introduced into the individual.
"Allogeneic" refers to a graft derived from a different animal of the same species.
"Xenogeneic" refers to a graft derived from an animal of a different species.
A "B cell surface marker" as used herein is an antigen expressed on the surface of a B cell which can be targeted with an agent which binds thereto. Exemplary B cell surface markers include the CD10, CD19, CD20, CD21, CD22, CD23, CD24, CD25, CD37, CD53, CD72, CD73, CD74, CD75, CD77, CD79a, CD79b, CD80, CD81, CD82, CD83, CD84, CD85 and CD86 leukocyte surface markers. The B cell surface marker of particular interest is preferentially expressed on B cells compared to other non-B cell tissues of a mammal and may be expressed on both precursor B cells and mature B cells. In one embodiment, the preferred marker is CD19, which is found on B cells throughout differentiation of the lineage from the pro/pre-B cell stage through the terminally differentiated plasma cell stage.
As used herein, "B cell depletion" refers to a reduction in B cell levels in an animal or human after drug, cellular or antibody treatment, as compared to the level before treatment. B cell levels are measurable using well known assays such as by getting a complete blood count, by FACS analysis staining for known B cell markers, and by methods described elsewhere herein. B cell depletion can be partial or complete. In one embodiment, the depletion of B cells is 25% or more.
The terms "deplete" and "depletion" are used herein in reference to B cells, and for purposes of the specification and claims, to mean one or more of: blocking of B cell function; functional inactivation of B cells; cytolysis of B cells; inhibiting the proliferation of B cells; inhibiting the differentiation of B cells to plasma cells; causing a B cell dysfunction which results in a therapeutic benefit; inhibiting production of anti-shed antigen antibody; reduction in the number of B cells; inactivation of B cells which have been primed or activated by shed antigen; blocking of one or more functions of B cells which have been primed or activated by shed antigen; cytolysis of B cells which have been primed or activated by shed antigen; and reduction in the number of B cells which have been primed or activated by shed antigen. B cell depletion may be a result of one or more mechanisms including, but not limited to, clonal inactivation, apoptosis, antibody-dependent cellular cytotoxicity, complement-mediated cytotoxicity, and a signal pathway mediated inactivation, dysfunction, or cell death.
The term "cancer" as used herein is defined as disease characterized by the rapid and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.
The "CD 19" antigen refers to an antigen of about 90 kDa which can be identified, for example, by the HD237 or B4 antibody (Kiesel et al., 1987 Leukemia Research II, 12:1119). CD19 is found on cells throughout differentiation of B-lineage cells from the stem cell stage through terminal differentiation into plasma cells, including but not limited to, pre-B cells, B cells (including naive B cells, antigen-stimulated B cells, memory B cells, plasma cells, and B lymphocytes) and follicular dendritic cells. CD19 is also found on B cells in human fetal tissue. In preferred embodiments, the CD19 antigen targeted by the antibodies of the invention is the human CD19 antigen.
"Co-stimulatory ligand," as the term is used herein, includes a molecule on an antigen presenting cell (e.g., an aAPC, dendritic cell, B cell, and the like) that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, proliferation, activation, differentiation, and the like. A co-stimulatory ligand can include, but is not limited to, CD7, B7-1 (CD80), B7-2 (CD86), PD-L1, PD-L2, 4-1BBL, OX40L, inducible costimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3. A co-stimulatory ligand also encompasses, *inter alia,* an antibody that specifically binds with a co-stimulatory molecule present on a T cell, such as, but not limited to, CD27, CD28, 4-1BB, OX40, CD30, CD40, PD-1, ICOS, lymphocyte function-associated antigen-1 (LFA-1), CD2, CD7, LIGHT, NKG2C, B7-H3, and a ligand that specifically binds with CD83.
A "co-stimulatory molecule" refers to the cognate binding partner on a T cell that specifically binds with a co-stimulatory ligand, thereby mediating a co-stimulatory response by the T cell, such as, but not limited to, proliferation. Co-stimulatory molecules include, but are not limited to an MHC class I molecule, BTLA and a Toll ligand receptor.
A "co-stimulatory signal," as used herein, refers to a signal, which in combination with a primary signal, such as TCR/CD3 ligation, leads to T cell proliferation and/or upregulation or downregulation of key molecules.
A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.
An "effective amount" as used herein, means an amount which provides a therapeutic or prophylactic benefit.
As used herein "endogenous" refers to any material from or produced inside an organism, cell, tissue or system.
As used herein, the term "exogenous" refers to any material introduced to an organism, cell, tissue or system that was produced outside the organism, cell, tissue or system.
The term "expression" as used herein is defined as the transcription and/or translation of a particular nucleotide sequence driven by its promoter.
"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (e.g., naked or contained in liposomes) and viruses (e.g., lentiviruses, retroviruses, adenoviruses, and adeno-associated viruses) that incorporate the recombinant polynucleotide.
"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared X 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.
The term "immunoglobulin" or "Ig," as used herein, is defined as a class of proteins, which function as antibodies. Antibodies expressed by B cells are sometimes referred to as the BCR (B cell receptor) or antigen receptor. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most subjects. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.
As used herein, the term "immune response" includes T cell mediated and/or B cell mediated immune responses. Exemplary immune responses include T cell responses, e.g., cytokine production and cellular cytotoxicity. In addition, the term immune response includes immune responses that are indirectly effected by T cell activation, e.g., antibody production (humoral responses) and activation of cytokine responsive cells, e.g., macrophages. Immune cells involved in the immune response include lymphocytes, such as B cells and T cells (CD4+, CD8+, Thl and Th2 cells); antigen presenting cells (e.g., professional antigen presenting cells such as dendritic cells, macrophages, B lymphocytes, Langerhans cells, and non-professional antigen presenting cells such as keratinocytes, endothelial cells, astrocytes, fibroblasts, oligodendrocytes); natural killer cells; myeloid cells, such as macrophages, eosinophils, mast cells, basophils, and granulocytes.
As used herein, the term "immunological tolerance" refers to methods performed on a proportion of treated subjects in comparison with untreated subjects where: a) a decreased level of a specific immunological response (thought to be mediated at least in part by antigen-specific effector T lymphocytes, B lymphocytes, antibody, or their equivalents); b) a delay in the onset or progression of a specific immunological response; or c) a reduced risk of the onset or progression of a specific immunological response. "Specific" immunological tolerance occurs when immunological tolerance is preferentially invoked against certain antigens in comparison with others.
As used herein, an "instructional material" includes a publication, a recording, a diagram, or any other medium of expression which can be used to communicate the usefulness of the compositions and methods of the invention. The instructional material of the kit of the invention may, for example, be affixed to a container which contains the nucleic acid, peptide, and/or composition of the invention or be shipped together with a container which contains the nucleic acid, peptide, and/or composition. Alternatively, the instructional material may be shipped separately from the container with the intention that the instructional material and the compound be used cooperatively by the recipient.
"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.
A "lentivirus" as used herein refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses. Vectors derived from lentiviruses offer the means to achieve significant levels of gene transfer in vivo.
By the term "modulating," as used herein, is meant mediating a detectable increase or decrease in the level of a response in a subject compared with the level of a response in the subject in the absence of a treatment or compound, and/or compared with the level of a response in an otherwise identical but untreated subject. The term encompasses perturbing and/or affecting a native signal or response thereby mediating a beneficial therapeutic response in a subject, preferably, a human.
"Parenteral" administration of an immunogenic composition includes, e.g., subcutaneous (s.c.), intravenous (i.v.), intramuscular (i.m.), or intrasternal injection, or infusion techniques.
The terms "patient," "subject," "individual," and the like are used interchangeably herein, and refer to any animal, or cells thereof whether in vitro or in situ, amenable to the methods described herein. In certain non-limiting embodiments, the patient, subject or individual is a human.
The term "rejection" refers to a state in which a transplanted organ or tissue is not accepted by the body of the recipient. Rejection results from the recipient's immune system attacking the transplanted organ or tissue. Rejection can occur days to weeks after transplantation (acute) or months to years after transplantation (chronic).
By the term "specifically binds," as used herein with respect to an antibody, is meant an antibody which recognizes a specific antigen, but does not substantially recognize or bind other molecules in a sample. For example, an antibody that specifically binds to an antigen from one species may also bind to that antigen from one or more species. But, such cross-species reactivity does not itself alter the classification of an antibody as specific. In another example, an antibody that specifically binds to an antigen may also bind to different allelic forms of the antigen. However, such cross reactivity does not itself alter the classification of an antibody as specific. In some instances, the terms "specific binding" or "specifically binding," can be used in reference to the interaction of an antibody, a protein, or a peptide with a second chemical species, to mean that the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A," the presence of a molecule containing epitope A (or free, unlabeled A), in a reaction containing labeled "A" and the antibody, will reduce the amount of labeled A bound to the antibody.
By the term "stimulation," is meant a primary response induced by binding of a stimulatory molecule (e.g., a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-β, and/or reorganization of cytoskeletal structures, and the like.
A "stimulatory molecule," as the term is used herein, means a molecule on a T cell that specifically binds with a cognate stimulatory ligand present on an antigen presenting cell.
A "stimulatory ligand," as used herein, means a ligand that when present on an antigen presenting cell (*e.g.,* an aAPC, a dendritic cell, a B-cell, and the like) can specifically bind with a cognate binding partner (referred to herein as a "stimulatory molecule") on a T cell, thereby mediating a primary response by the T cell, including, but not limited to, activation, initiation of an immune response, proliferation, and the like. Stimulatory ligands are well-known in the art and encompass, inter alia, an MHC Class I molecule loaded with a peptide, an anti-CD3 antibody, a superagonist anti-CD28 antibody, and a superagonist anti-CD2 antibody.
The term "subject" is intended to include living organisms in which an immune response can be elicited (e.g., mammals). Examples of subjects include humans, dogs, cats, mice, rats, and transgenic species thereof.
As used herein, a "substantially purified" cell is a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some embodiments, the cells are cultured *in vitro.* In other embodiments, the cells are not cultured *in vitro.*
The term "therapeutic" as used herein means a treatment and/or prophylaxis. A therapeutic effect is obtained by suppression, remission, or eradication of a disease state.
The term "therapeutically effective amount" refers to the amount of the subject compound that will elicit the biological or medical response of a tissue, system, or subject that is being sought by the researcher, veterinarian, medical doctor or other clinician. The term "therapeutically effective amount" includes that amount of a compound that, when administered, is sufficient to prevent development of, or alleviate to some extent, one or more of the signs or symptoms of the disorder or disease being treated. The therapeutically effective amount will vary depending on the compound, the disease and its severity and the age, weight, etc., of the subject to be treated.
A "transplant," as used herein, refers to cells, tissue, or an organ that is introduced into an individual. The source of the transplanted material can be cultured cells, cells from another individual, or cells from the same individual (e.g., after the cells are cultured in vitro). Exemplary organ transplants are kidney, liver, heart, lung, and pancreas.
To "treat" a disease as the term is used herein, means to reduce the frequency or severity of at least one sign or symptom of a disease or disorder experienced by a subject.
The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.
The term "tolerant" refers to an individual with a reduced or absent immune response to a specific antigen or group of antigens. In the context of the invention, an individual is considered tolerant if he or she does not reject (i.e., mount a significant immune response against) transplanted cells. In some cases, the tolerant individual does not reject transplanted cells, even in the absence of immunosuppressive therapy. In the context of the invention, an individual is considered "non-tolerant" if the individual rejects transplanted cells. Non-tolerant individuals include those where rejection is controlled using immunosuppressive therapy (e.g., standard immunosuppression), as well as those that are experiencing an active immune response against transplanted cells.
As used herein, "in vivo tolerance" refers to the substantial lack of immune response specific for the foreign tissue. The immune response may stem from the recipient subject mounting an immune response to a foreign tissue, or conversely, the immune response may stem from the foreign tissue mounting an immune response to the recipient subject (e.g. GVHD). Methods of measuring in vivo tolerance are commonly known in the art.
Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The present disclosure describes compositions and methods for depleting normal B cells in a mammal. In one embodiment, depletion of B cells using the CAR of the invention induces tolerance in the mammal.

In one embodiment, the present disclosure provides a method of inducing in vivo tolerance to transplanted foreign tissue. In some embodiments, the method may be used, in part, to prevent and/or treat the rejection of a transplanted tissue. Generally speaking, the method comprises administering a CAR T cell of the invention to a subject exposed to transplanted foreign tissue. The term "foreign tissue," as used herein, may encompass a bone marrow transplant, an organ transplant, a blood transfusion, or any other foreign tissue or cell that is purposefully introduced into a subject.

In another embodiment, the method may be used, in part, to prevent and/or treat graft-versus-host disease (GVHD). Generally speaking, the method comprises administering a CAR T cell of the invention to a subject exposed to transplanted foreign tissue. The term "foreign tissue," as used herein, may encompass a bone marrow transplant, an organ transplant, a blood transfusion, or any other foreign tissue or cell that is purposefully introduced into a subject.

In one embodiment, the CAR described herein can be engineered to comprise an extracellular domain having an antigen binding domain that targets a B cell antigen fused to an intracellular signaling domain of the T cell antigen receptor complex zeta chain (e.g., CD3 zeta). An exemplary B cell antigen is CD19 because this antigen is expressed on malignant B cells. However, the disclosure is not limited to targeting CD19. Rather, the disclosure includes any B cell antigen binding moiety that when bound to its cognate antigen. The antigen binding moiety is preferably fused with an intracellular domain from one or more of a costimulatory molecule and a zeta chain. Preferably, the antigen binding moiety is fused with one or more intracellular domains selected from the group of a CD137 (4-1BB) signaling domain, a CD28 signaling domain, a CD3zeta signal domain, and any combination thereof.

In one embodiment, the CAR described herein comprises a CD137 (4-1BB) signaling domain. This is because the present invention is partly based on the discovery that CAR-mediated T-cell responses can be further enhanced with the addition of costimulatory domains. For example, inclusion of the CD137 (4-1BB) signaling domain significantly increased CAR mediated activity and *in vivo* persistence of CAR T cells compared to an otherwise identical CAR T cell not engineered to express CD137 (4-1BB). However, the disclosure is not limited to a specific CAR. Rather, any CAR that targets a B cell can be used in the present invention. Compositions and methods of making CARs have been described in WO 2012/079000.

### Methods

The disclosure relates to methods of using the CAR and CAR T cells described herein to deplete B cells and to promote tolerance. In one embodiment, the method includes promoting transplantation tolerance (e.g., of organ or tissue transplants) in a patient. In another embodiment, the method includes the prevention and/or treatment of GVHD. In a specific embodiment, the CAR described herein targets CD19 on B cells.

In one embodiment, the ability to induce sustained donor humoral tolerance is a key to achieving robust transplantation tolerance and/or preventing or treating GVHD. The disclosure encompasses the use of the CAR T cells described herein invention to deplete B cells and to induce tolerance by administering the CAR T cells to an animal, preferably a mammal, and most preferably a human, patient for treating one or more diseases, disorders, symptoms, or conditions associated with organ or tissue transplant (e.g., transplant rejection, GVHD and/or conditions associated therewith).

Organ rejection occurs by host immune cell destruction of the transplanted tissue through an immune response. Similarly, an immune response is also involved in GVHD, but, in this case, the foreign transplanted immune cells destroy the host tissues. For example, organ rejection and/or GVHD may occur after heart, heart valve, lung, kidney, liver, pancreas, intestine, skin blood vessel, bone marrow, stem cell, bone, or islet cell transplantation. However, the invention is not limited to a specific type of transplantation. By way of a non-limiting example, an islet cell transplantation can be performed to prevent the onset of diabetes or as a treatment of diabetes. The administration of the CAR T cells described herein that inhibit an immune response, particularly the proliferation, differentiation, or survival of B-cells, is an effective therapy in preventing organ and/or tissue rejection or GVHD. The administration of CAR T cells described herein also can be used to promote transplantation tolerance following organ and/or tissue transplantation.

The CAR T cells described herein can also be used to promote transplantation tolerance; to treat, decrease, inhibit and/or prevent the rejection of organ and/or tissue transplants; and/or to decrease antibody titer in a patient who has received an organ or tissue transplant. In one embodiment, the CAR T cells described herein can be used to promote transplantation tolerance in a patient by administering to the patient an effective amount of the CAR T cells described herein, thereby preventing or delaying transplant rejection. In another embodiment, the CAR T cells described herein can be used to treat organ or transplant rejection in a patient by administering to the patient an effective amount of the CAR T cells described herein,
thereby inhibiting transplant organ or tissue rejection. In yet another embodiment, the CAR T cells described herein can be used to decrease antibody titer in a patient who has received, or will receive, an organ or tissue transplant by administering to the patient an effective amount of the CAR T cells described herein, thereby decreasing antibody titer.

In one embodiment, the disclosure provides a method of promoting transplantation tolerance in a patient comprising administering to the patient an effective amount of the CAR T cells described herein thereby delaying transplant rejection in the patient.

In another embodiment, the disclosure provides a method of treating transplant organ or tissue rejection in a patient comprising administering to the patient an effective amount of the CAR T cells described herein, thereby inhibiting transplant organ or tissue rejection in the patient.

In another embodiment, the disclosure provides a method of decreasing antibody titer in a patient who has received, or will received, an organ or tissue transplant comprising administering to the patient an effective amount of the CAR T cells described herein, thereby decreasing antibody titer in the patient.

In one embodiment, the disclosure provides a method of inhibiting or reducing immunoglobulin production in a patient comprising administering to the patient an effective amount of the CAR T cells described herein.

In one embodiment, the CAR T cells described herein decrease or inhibit B cell function. In another embodiment, the CAR T cells described herein deplete or eliminate B cells from the subject. For example, the CAR T cells described herein invention can be engineered to target a B cell surface antigen in order to allow the T cell to exhibit effector functions against the B cell.

### Therapy to Inhibit Adverse Immune Responses Following Transplantation

The present invention includes using CAR T cells described herein as a therapy to inhibit GVHD or graft rejection following transplantation. Accordingly, the present disclosure encompasses a method of contacting a donor transplant, for example a biocompatible lattice or a donor tissue, organ or cell, with CAR T cells described herein prior to, concurrently with, or after transplantation of the transplant into a recipient. The CAR T cells described herein serve to ameliorate, inhibit or reduce an adverse response by the donor transplant against the recipient, thereby preventing or treating GVHD.

As discussed elsewhere herein, T cells can be obtained from any source, for example, from the tissue donor, the transplant recipient or an otherwise unrelated source (a different individual or species altogether) for generation of CAR T cells described herein for the use of eliminating or reducing an unwanted immune response by a transplant against a recipient of the transplant. Accordingly, CAR T cells described herein can be autologous, allogeneic or xenogeneic to the tissue donor, the transplant recipient or an otherwise unrelated source.

In an embodiment of the present disclosure, the transplant is exposed to the CAR T cells described herein prior, at the same time, or after transplantation of the transplant into the recipient. In this situation, an immune response against the transplant caused by any alloreactive recipient cells would be suppressed by the CAR T cells described herein present in the transplant because the CAR T cells can deplete B cells and induce tolerance.

In another embodiment of the present disclosure, the donor transplant can be "preconditioned" or "pretreated" by treating the transplant prior to transplantation into the recipient in order to reduce the immunogenicity of the transplant against the recipient, thereby reducing and/or preventing GVHD or graft rejection. The transplant can be contacted with cells or a tissue from the recipient prior to transplantation in order to activate T cells that may be associated with the transplant. Following the treatment of the transplant with cells or a tissue from the recipient, the cells or tissue may be removed from the transplant. The treated transplant is then further contacted with CAR T cells described herein in order to reduce, inhibit or eliminate the activity of the T and/or B cells that were activated by the treatment of the cells or tissue from the recipient. Following this treatment of the transplant with CAR T cells described herein, the CAR T cells may be removed from the transplant prior to transplantation into the recipient. However, some CAR T cells may adhere to the transplant, and therefore, may be introduced to the recipient with the transplant. In this situation, the CAR T cells introduced into the recipient can suppress an immune response against the recipient caused by any cell associated with the transplant. Without wishing to be bound to any particular theory, the treatment of the transplant with CAR T cells prior to transplantation of the transplant into the recipient serves to reduce, inhibit or eliminate the activity of the activated T and/or B cells, thereby preventing restimulation, or inducing hyporesponsiveness of the T and/or cells to subsequent antigenic stimulation from a tissue and/or cells from the recipient. One skilled in the art would understand based upon the present disclosure, that preconditioning or pretreatment of the transplant prior to transplantation may reduce or eliminate the graft versus host response.

### Therapeutic Application

In one embodiment, the present disclosure includes a type of cellular therapy where T cells are genetically modified to express a CAR and the CAR T cell is infused to a recipient in need thereof. The infused cell is able to kill a targeted cell. In one embodiment, the targeted cell is a B cell. Unlike antibody therapies, CAR T cells are able to replicate *in vivo* resulting in long-term persistence that can lead to sustained B cell depletion and tolerance.

In one embodiment, the CAR T cells described herein can undergo robust *in vivo* T cell expansion and can persist for an extended amount of time. In another embodiment, the CAR T cells described herein evolve into specific memory T cells that can be reactivated to inhibit B cell proliferation. For example, a CART19 cells elicits an immune response specific against cells expressing CD19.

The CAR-modified T cells described herein may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cell into a mammal: i) expansion of the cells, ii) introducing a nucleic acid encoding a CAR to the cells, and/or iii) cryopreservation of the cells.

*Ex vivo* procedures are well known in the art and are discussed more fully below. Briefly, cells are isolated from a mammal (preferably a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of *ex vivo* immunization, the present disclosure also provides compositions and methods for *in vivo* immunization to elicit an immune response directed against a B cell antigen in a patient.

Generally, the cells activated and expanded as described herein may be utilized in the depletion of B cells and induction of tolerance. In particular, the CAR-modified T cells described herein are used in the treatment of one or more diseases, disorders, symptoms, or conditions associated with organ or tissue transplant (e.g., GVHD and/or conditions associated therewith). Thus, the present disclosure provides methods for the treatment or prevention of organ rejection and GVHD comprising administering to a subject in need thereof, a therapeutically effective amount of the CAR-modified T cells of the described herein.

In one embodiment, the CAR T cells described herein are administered in conjunction with an immunosuppressant agent. Any immunosuppressant agent known in the art may be used. For example, the immunosuppressant agent may be Cyclosporine, Azathioprine, Rapamycin, Mycophenolate mofetil, Mycophenolic acid, Prednisone, Sirolimus, Basiliximab, or Daclizumab, or any combination thereof. Additional specific immunosuppressants that may be used include, but are not limited to, ORTHOCLONE OKT™ 3 (muromonab-CD3), SANDIMMUNE™, NEORAL™, SANGDYA™ (cyclosporine), PROGRAF™ (FK506, tacrolimus), CELLCEPT™ (mycophenolate motefil, of which the active metabolite is mycophenolic acid), IMURAN™ (azathioprine), glucorticosteroids, adrenocortical steroids such as DELTASONE™ (prednisone) and HYDELTRASOL™ (prednisolone), FOLEX™ and MEXATE™ (methotrxate), OXSORALEN-ULTRA™ (methoxsalen), RITUXAN™ (rituximab), and RAPAMUNE™ (sirolimus).

The CAR T cells described herein can be administered to the patient before, after, or concomitant with the immunosuppressant agent. For example, the CAR T cells described herein can be administered after the immunosuppressant agent is administered to the patient or the CAR T cells described herein can be administered before the immunosuppressant agent is administered to the patient. Alternatively, or in addition, the CAR T cells described herein are administered at the same time the immunosuppressant agent is administered to the patient.

The CAR T cells described herein and/or the immunosuppressant agent can be administered to the patient after transplantation. Alternatively, or in addition, the CAR T cells described herein and/or the immunosuppressant agent can be administered to the patient before transplantation. The CAR T cells described herein and/or the immunosuppressant agent also can be administered to the patient during transplantation surgery.

In some embodiments, the method described herein of administering CAR T cells to the patient is carried out once immunosuppressive therapy has been initiated. In some embodiments, the method is carried out more than once, e.g., to monitor the transplant recipient over time, and, if applicable, in different immunosuppressive therapy regimes. In some embodiments, immunosuppressive therapy is reduced if the transplant recipient is predicted to be tolerant of the transplant. In some embodiments, no immunosuppressive therapy is prescribed, e.g., immunosuppressive therapy is ceased, if the transplant recipient is predicted to be tolerant of the transplant. If the transplant recipient demonstrates a non-tolerant biomarker signature, immunosuppressive therapy can be restored to or continued at a standard level.

The organ or tissue transplant may be a heart, heart valve, lung, kidney, liver, pancreas, intestine, skin, blood vessels, bone marrow, stem cells, bone, or, islet cells.

The CAR T cells described herein can be administered following a diagnosis of transplant organ or tissue rejection followed by doses of both the CAR T cells described herein and an immunosuppressant agent until symptoms of organ or tissue rejection subside.

In some embodiments, the CAR T cells described herein is administered following a diagnosis of increased antibody titer followed by doses of both the CAR T cells described herein and the immunosuppressant agent until antibody titer decreases.

Preferably, treatment using the CAR T cells described herein is accomplished by administering an effective amount of CAR T cells described herein to the patient.

The CAR T cells described herein may be administered either alone, or as a pharmaceutical composition in combination with diluents and/or with other components such as IL-2 or other cytokines or cell populations. Briefly, pharmaceutical compositions described herein may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e.g.*, aluminum hydroxide); and preservatives. Compositions described herein are preferably formulated for intravenous administration.

Pharmaceutical compositions described herein may be administered in a manner appropriate to the disease to be treated (or prevented). The quantity and frequency of administration will be determined by such factors as the condition of the patient, and the type and severity of the patient's disease, although appropriate dosages may be determined by clinical trials.

When the "effective amount" is indicated, the precise amount of the compositions described herein : administered can be determined by a physician with consideration of individual differences in age, weight, antibody titer, and condition of the patient (subject). It can generally be stated that a pharmaceutical composition comprising the T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, *e.g.,* Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

In certain embodiments, it may be desired to administer activated T cells to a subject and then subsequently redraw blood (or have an apheresis performed), activate T cells therefrom according to the present invention, and reinfuse the patient with these activated and expanded T cells. This process can be carried out multiple times every few weeks. In certain embodiments, T cells can be activated from blood draws of from 10cc to 400cc. In certain embodiments, T cells are activated from blood draws of 20cc, 30cc, 40cc, 50cc, 60cc, 70cc, 80cc, 90cc, or 100cc. Not to be bound by theory, using this multiple blood draw/multiple reinfusion protocol may serve to select out certain populations of T cells.

The administration of the subject compositions may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous (*i.v.*) injection, or intraperitoneally. In one embodiment, the T cell compositions described herein are administered to a patient by intradermal or subcutaneous injection. In another embodiment, the T cell compositions described herein are preferably administered by *i.v.* injection. The compositions of T cells may be injected directly into a tumor, lymph node, or site of infection.

In certain embodiments described herein, cells activated and expanded using the methods described herein, or other methods known in the art where T cells are expanded to therapeutic levels, are administered to a patient in conjunction with (*e.g.*, before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells described herein may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, cytoxin, fludaribine, cyclosporin, FK506, rapamycin, mycophenolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin) (Liu et al., Cell 66:807-815, 1991; Henderson et al., Immun. 73:316-321, 1991; Bierer et al., Curr. Opin. Immun. 5:763-773, 1993). In a further embodiment, the cell compositions described herein are administered to a patient in conjunction with (*e.g.*, before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In another embodiment, the cell compositions described herein are administered following B-cell ablative therapy such as agents that react with CD20, *e.g.,* Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

### EXPERIMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### Example 1: T cells expressing chimeric receptors deplete normal B cells and induce tolerance

The results presented herein demonstrate that that CART 19 cells persist and provide a therapeutic benefit in the patient for at least 18 months. The engineered T cells expanded more than a thousand-fold *in vivo,* trafficked to bone marrow and continued to express functional CARs at high levels for at least 6 months. On average, each infused CAR+ T cell eradicated at least 1000 CLL cells. A CD19 specific immune response was demonstrated in the blood and bone marrow, accompanied by complete remission in two of three patients. A portion of the cells persist as memory CAR+ T cells, indicating the potential of this non-MHC restricted approach for the effective treatment of B cell malignancies.

The materials and methods employed in these experiments are now described.

### Materials and Methods

### Protocol Design

The clinical trial (NCT01029366) was conducted as described in WO 2012/079000.

### Vector Production

The CD19-BB-z transgene (GeMCRIS 0607-793) was designed and constructed as described (Milone et al., 2009, Mol Ther. 17:1453-1464). Lentiviral vector was produced according to current good manufacturing practices using a three-plasmid production approach at Lentigen Corporation as described (Zufferey et al., 1997, Nature biotechnol 15:871-875).

### Preparation of CART19 cell product

Methods of T cell preparation using paramagnetic polystyrene beads coated with anti-CD3 and anti-CD28 monoclonal antibodies have been described (Laport et al., 2003, Blood 102: 2004-2013). Lentiviral transduction was performed as described (Levine et al., 2006, Proc Natl Acad Sci U S A 103:17372-17377).

The results of the experiments are now described.

### In vivo expansion and persistence of CART19 and trafficking to bone marrow

CAR+ T cells expanded using CD3/CD28 beads and expressing a 4-1BB signaling domain is believed to be in improvement to CARs lacking 4-1BB. A Q-PCR assay was developed to enable quantitative tracking of CART19 cells in blood and bone marrow. All patients had expansion and persistence of the CART19-cells in blood for at least 6 months as depicted in Figures 1A and 1C. Notably, patients UPN 01 and UPN 03 had a 1,000 to 10,000 fold expansion of CAR+ T cells in blood during the first month post infusion. The peak expansion levels coincided with onset of the post-infusion clinical symptoms in patient UPN 01 (day 15) and patient UPN 03 (day 23). Furthermore, following an initial decay that can be modeled with first order kinetics, the CART19 T cell levels stabilized in all 3 patients from day 90 to 180 post infusion. Significantly, the CART19 T cells also trafficked to bone marrow in all patients, albeit at 5-to 10-fold lower levels than observed in blood as depicted in Figures ID through IF. Patients UPN 01 and 03 had a log linear decay in the marrow, with a disappearance T½ of ∼35 days.

### Prolonged expression and establishment of a population of memory CART19 cells in blood

A central question in CAR-mediated cancer immunotherapy is whether optimized cell manufacturing and costimulation domains enhance the persistence of genetically modified T cells and permit the establishment of CAR+ memory T cells in patients. Previous studies have not demonstrated robust expansion, prolonged persistence and/or expression of CARs on T cells after infusion (Kershaw et al., 2006, Clin Cancer Res 12:6106-6115; Lamers et al., 2006, J Clin Oncol 24:e20-e22; Till et al., 2008, Blood, 112, 2261-2271; Savoldo et al., 2011, J Clin Invest doi:10.1172/JCI46110). Flow-cytometric analysis of samples from both blood and marrow at 169 days post infusion revealed the presence of CAR19 expressing cells in UPN 03 (Figures 2A and 2B), and an absence of B cells as depicted in Figure 2A. Notably, by Q-PCR assay, all three patients have persisting CAR+ cells at 4 months and beyond as depicted in Figures 1 and Figures 4. The *in vivo* frequency of CAR+ cells by flow cytometry closely matched the values obtained from the PCR assay for the CART19 transgene. Importantly, in patient UPN 03, only CD3+ cells expressed the CAR19, as CAR19+ cells were not detectable in CD16-or CD14-positive subsets as depicted in Figure 2A. CAR expression was also detected on the surface of 4.2% of T cells in the blood of patient UPN 01 on day 71 post infusion as depicted in Figure 5.

Next, polychromatic flow cytometry was used to perform detailed studies to further characterize the expression, phenotype, and function of CART19 cells in UPN 03 using an anti-CAR idiotype antibody (MDA-647) and a gating strategy shown in Figure 6. Notable differences in the expression of memory and activation markers in both CD8+ and CD4+ cells based on CAR19 expression was observed. At day 56, CART19 CD8+ cells displayed primarily an effector memory phenotype (CCR7-CD27-CD28-) consistent with prolonged and robust exposure to antigen as depicted in Figure 2C. In contrast, CAR-negative CD8+ cells consisted of mixtures of effector and central memory cells, with CCR7 expression in a subset of cells, and substantial numbers in the CD27+/CD28-and CD27+/CD28+ fractions. While both CART19 and CAR-negative cell populations substantially expressed CD57, this molecule was uniformly co-expressed with PD-1 in the CART19 cells, a possible reflection of the extensive replicative history of these cells. In contrast to the CAR-negative cell population, the entirety of the CART19 CD8+ population lacked expression of both CD25 and CD127. By day 169, while the phenotype of the CAR-negative cell population remained similar to the day 56 sample, the CART19 population had evolved to contain a minority population with features of central memory cells, notably expression of CCR7, higher levels of CD27 and CD28, as well as CAR+ cells that were PD-1-negative, CD57-negative and CD127-positive.

In the CD4+ compartment, at day 56 CART19 cells were characterized by uniform lack of CCR7 and a predominance of CD27+/CD28+/PD-1+ cells distributed within both CD57+ and -compartments, and an essential absence of CD25 and CD 127 expression as depicted in Figure 2B. In contrast, CAR-negative cells at this time-point were heterogeneous in CCR7, CD27 and PD-1 expression, expressed CD127 and also contained a substantial CD25+/CD127-(potential regulatory T cell) population. By day 169, while CD28 expression remained uniformly positive in all CAR+CD4+ cells, a fraction of the CART19 CD4+ cells had evolved toward a central memory phenotype with expression of CCR7, a higher percentage of CD27-cells, the appearance of a PD-1-negative subset, and acquisition of CD127 expression. CAR-negative cells remained reasonably consistent with their day 56 counterparts, with the exception of a reduction in CD27 expression a decrease in the percentage of CD25+/CD127-cells.

### CART19 cells can retain effector function after 6 months in blood

In addition to short persistence and inadequate in vivo proliferation, a limitation of previous trials with CAR+ T cells has been the rapid loss of functional activity of the infused T cells *in vivo.* The high level CART19 cell persistence and surface expression of the CAR19 molecule in patient UPN 01 and 03 provided the opportunity to directly test anti-CD 19-specific effector functions in cells recovered from cryopreserved peripheral blood samples. PBMC from patient UPN 03 were cultured with target cells that were either positive or negative for CD19 expression. Robust CD 19-specific effector function of CART 19 T cells was demonstrated by specific degranulation against CD19-positive but not CD19-negative target cells, as assessed by surface CD107a expression. Notably, exposure of the CART19 population to CD19-positive targets induced a rapid internalization of surface CAR-19 as depicted in Figure 6 for surface expression of CAR19 in the same effector cells in standard flow-cytometric staining. The presence of
costimulatory molecules on target cells was not required for triggering CART19 cell degranulation because the NALM-6 line does not express CD80 or CD86 (Brentjens et al., 2007, Clin Cancer Res 13:5426-5435). Effector function was evident at day 56 post infusion and was retained at the day 169 time-point. Robust effector function of CAR+ and CAR-T cells could also be demonstrated by pharmacologic stimulation.

### Clinical activity of CART19 cells

There were no significant toxicities observed during the four days following the infusion in any patient, other than transient febrile reactions. However, all patients subsequently developed significant clinical and laboratory toxicities between day 7 and 21 following the first infusion. These toxicities were short-term and reversible. Of the three patients treated to date, there are 2 CRs and 1 PR at >6 months post CART19 infusion according to standard criteria (Hallek et al., 2008, Blood 111:5446). Details of past medical history and response to therapy for each patient are depicted in Figure 7.

In brief, patient UPN 01 developed a febrile syndrome, with rigors and transient hypotension beginning 10 days after infusion. The fevers persisted for approximately 2 weeks and resolved; the patient has had no further constitutional symptoms. The patient achieved a rapid and complete response as depicted in Figure 3. Between 1 and 6 months after infusion, no circulating CLL cells have been detected in the blood by flow cytometry. Bone marrow at 1, 3, and 6 months after CART19 cell infusions shows sustained absence of the lymphocytic infiltrate by morphology and flow cytometric analysis as depicted in Figure 3B. CT scans at 1 and 3 months after infusion show resolution of adenopathy as depicted in Figure 3C. Complete remission was sustained for 10+ months at the time of this report.

Patient UPN 02 was treated with 2 cycles of bendamustine with rituximab resulting in stable disease as depicted in Figure 3A. The patient received a third dose of bendamustine as lymphodepleting chemotherapy prior to CART19 T cell infusion. The patient developed fevers to 40°C, rigors and dyspnea requiring a 24 hour hospitalization on day 11 after the first infusion and on the day of the second CART19 cell boost. Fevers and constitutional symptoms persisted and on day 15, the patient had transient cardiac dysfunction; all symptoms resolved after corticosteroid therapy was initiated on day 18. Following CART19 infusion, and coincident with the onset of high fevers, the patient had rapid clearance of the p53-deficient CLL cells from peripheral blood as depicted in Figure 3A and a partial reduction of adenopathy, bone marrow showed persistent extensive infiltration of CLL one month after therapy despite dramatic peripheral blood cytoreduction. The patient remains asymptomatic.

Patient UPN 03 received pentostatin and cyclophosphamide as lymphodepleting chemotherapy prior to CART19 cell infusion. Three days after chemotherapy but prior to cell infusion, bone marrow was hypercellular (60%) with approximately 50% involvement by CLL. The patient received a low dose of CART19 cells (1.5x10⁵ CAR+ T cells/kg divided over 3 days). Again, there were no acute infusional toxicities. However, 14 days after the first infusion, the patient began having rigors, fevers, nausea and diarrhea. By day 22 after infusion, tumor lysis syndrome was diagnosed requiring hospitalization. The patient had resolution of constitutional symptoms, and within 1 month of CART19 infusions, the patient had clearance of circulating CLL from the blood and bone marrow by morphology, flow cytometry, cytogenetic, and FISH analysis. CT scans showed resolution of abnormal adenopathy as depicted in Figures 3B and 3C. Complete remission was sustained beyond 8 months from the initial CART19 cell infusion.

### Considerations of in vivo CART19 effector to CLL target cell ratio

Pre-clinical studies showed that large tumors could be ablated, and that the infusion of 2.2x10⁷CARs could eradicate tumors comprised of 1x10⁹ cells, for an *in vivo* E:T ratio of 1:42 in humanized mice (Carpenito et al., 2009, Proc Natl Acad Sci USA 106:3360-3365), although these calculations did not take into account the expansion of T cells after injection. Estimation of CLL tumor burden over time permitted the calculation of tumor reduction and the estimated CART 19 E:T ratios achieved *in vivo* in the three subjects based on number of CAR+ T cells infused. Tumor burdens were calculated by measuring CLL load in bone marrow, blood and secondary lymphoid tissues. The baseline tumor burdens as shown in Figure 7 indicate that each patient had on the order of 10¹² CLL cells (i.e., 1 kilogram tumor load) before CART19 infusion. Patient UPN 03 had an estimated baseline tumor burden of 8.8x10¹¹ CLL cells in the bone marrow on day -1 (i.e. post chemotherapy and pre-CART19 infusion), and a measured tumor mass in secondary lymphoid tissues of 3.3 -5.5x10¹¹CLL cells, depending on the method of volumetric CT scan analysis. Given that UPN 03 was infused with only 1.4x10⁷CART19 cells, using the estimate of initial total tumor burden (1.3x10¹² CLL cells), and that no CLL cells are detectable post treatment, a striking 1:93,000 E:T ratio was achieved. By similar calculations, an effective E:T ratio *in vivo* of 1:2200 and 1:1000 was calculated for UPN 01 and UPN 02. In the end, a contribution of serial killing by CART19 T cells, combined with *in vivo* CART19 expansion of > 1,000-fold likely contributed to the powerful anti-leukemic effects mediated by CART19 cells.

### T cells expressing chimeric receptors establish memory and potent antitumor effects in patients with advanced leukemia

Limited *in vivo* expression and effector function of CARs has been a central limitation in the trials testing first generation CARs (Kershaw et al., 2006, Clin Cancer Res 12:6106-6115; Lamers et al., 2006, J Clin Oncol 24:e20-e22; Till et al., 2008, Blood, 112, 2261-2271; Park et al., 2007, Mol Ther 15:825833; Pule et al., 2008, Nat Med 14:1264-1270). Based on pre-clinical modeling demonstrating enhanced persistence of CARs containing a 4-1BB signaling module (Milone et al., 2009, Mol Ther. 17:1453-1464; Carpenito et al., 2009, Proc Natl Acad Sci USA 106:3360-3365), experiments were designed to develop a second generation of CARs engineered with lentiviral vector technology. This second generation of CARs was found to be safe in the setting of chronic HIV infection (Levine et al., 2006, Proc Natl Acad Sci U S A 103:17372-17377). The present results show that when this second generation CAR was expressed in T cells and cultured under conditions designed to promote engraftment of central memory T cells (Rapoport et al., 2005, Nat Med 11:1230-1237; Bondanza et al., 2006, Blood 107:1828-1836), improved expansion of CAR T cells after infusion was observed compared to previous reports. CART19 cells established CD19-specific cellular memory, and killed tumor cells at E:T ratios *in vivo* not previously achieved.

CART19 is the first CAR trial to incorporate a 4-1BB signaling domain and the first to use lentiviral vector technology. The present results demonstrate efficient tracking of CARs to sites of tumor, with the *de facto* establishment of "tumor infiltrating lymphocytes" that exhibited CD19 specificity. The pronounced *in vivo* expansion permitted the first demonstration that CARs directly recovered from patients can retain effector function *in vivo* for months. A previous study had suggested that introduction of a first generation CAR into virus specific T cells is preferable to primary T cells (Pule et al., 2008, Nat Med 14:1264-1270), however the results with second generation CARs introduced into optimally costimulated primary T cells calls this notion into question. Without wishing to be bound by any particular theory, a cautionary note is raised that the clinical effects were profound and unprecedented with the lysis of kilogram sized tumor burdens in all three patients accompanied with the delayed release of potentially dangerously high levels of cytokines in two of the patients. Classical cytokine storm effects were not observed. However, the present study was designed to mitigate this possibility by deliberate infusion of CART 19 over a period of three days.

It was found that very low doses of CARs can elicit potent clinical responses. This was a pilot study that demonstrated safety of the CART 19 vector design. The observation that doses of CART19 cells several orders of magnitude below those tested in previous trials can have clinical benefit may have important implications for future implementation of CAR therapy on a wider scale, and for the design of trials testing CARs directed against targets other than CD 19.

The present studies further indicate that CART19 is expressed in both central memory and effector T cells, and this likely contributes to their long term survival compared to previous reports. Without wishing to be bound by any particular theory, CAR T cells may differentiate *in vivo* into a central memory-like state upon encounter and subsequent elimination of target cells (e.g. CLL tumor cells or normal B cells) expressing the surrogate antigen. Indeed signaling of 4-1BB has been reported to promote the development of memory in the context of TCR signaling (Sabbagh et al., 2007, Trends Immunol 28:333-339).

The extended proliferation and survival of CART19 has revealed aspects of the pharmacokinetics of CAR T cells that have not previously been reported. It was observed that the kinetics of cytokine release in serum and marrow correlated with peak CART19 levels, so that it is possible that the decay is initiated when cellular targets expressing CD 19 become limiting. The mechanism of the extended survival of CART19 may relate to the aforementioned incorporation of the 4-1BB domain or to signaling through the natural TCR and/or CAR. An intriguing possibility is that the extended survival is related to the population of CART 19 that has been identified in marrow specimens, raising the hypothesis that CD19 CARs could be maintained by encounter with B cell progenitors in the bone marrow. Related to this question is what drives the initial expansion of CART19 cells *in vivo?* With rare exceptions (Savoldo et al., 2011, J Clin Invest doi: 10.1172/JCI46110; Pule et al., 2008, Nat Med 14:1264-1270), the present study is the only trial to have omitted IL-2 infusions, so that the CART19 cells likely either expanded in response to homeostatic cytokines or more likely, to CD19 expressed on leukemic targets and/or normal B cells. In the latter case, this could be an attractive feature for CARs directed against targets on normal APCs such as CD19 and CD20, as it is possible that self-renewal of CART19 occurs on the normal cells, providing a mechanism for CAR memory by means of "self vaccination/boosting" and therefore, long term tumor immunosurveillance. The mechanisms of CART19 homeostasis may require further study to elucidate cell intrinsic and extrinsic mechanisms of persistence. Previous to these results, most investigators have viewed CAR therapy as a transient form of immunotherapy, however CARs with optimized signaling domains may have a role in both remission induction and consolidation as well as for long term immunosurveillance.

Potent anti-leukemic effects have been observed in all three patients, including two patients with p53 deficient leukemia. Previous studies with CARs have had difficulty separating antitumor effects from lymphodepleting chemotherapy. However, the delayed cytokine release combined with the kinetics of tumor lysis in fludarabine-refractory patients that was coincident, and possibly dependent on *in vivo* CAR expansion in the present study, indicate that CART19 mediates potent antitumor effects. The present results do not exclude a role for chemotherapy in potentiating the effects of CARs.

A thorough comparison of the vector, transgene and cell manufacturing procedures with results from ongoing studies at other centers may be required to gain a full understanding of the key features required to obtain sustained function of CAR T cells *in vivo.* Unlike antibody therapies, CAR-modified T cells have the potential to replicate *in vivo,* and long-term persistence could lead to sustained tumor control. The availability of an off the shelf therapy comprised of non-cross resistant killer T cells has the potential to improve the outcome of patients with B cell malignancies. A limitation of antibody therapy, as for example, with agents such as rituximab and bevicizumab, is that the therapy requires repeated antibody infusions, that is inconvenient and costly. The delivery of prolonged antibody therapy (in this case for at least 6 months in 3 of 3 patients treated to date) with anti-CD 19 scFv expressed on T cells following a single infusion of CART19 cells has a number of practical advantages, including conveniences and cost savings.

### Sustained detection of CART 19 eighteen months post-infusion

The results presented herein show long term expression of CART19 and deep B cell aplasia (Figures 8 and 9), and a reduction in plasma cells in all 3 patients (Figure 10). A major surprise from the CART19 trial was that the CART19 cells having a murine scFv that exhibited highly immunogenic phenotypes were in fact not rejected by the immune system of the host patient. This suggests that the CART 19 cells depleted normal B cells in the host patient and as a result induced tolerance.

Without wishing to be bound by any particular theory, CART 19 cells can be used for the following applications: 1) solid organ transplant patients who are "cross match" positive; elimination of pre-existing memory B cells might permit organ transplants that are not currently possible in these immunized patients; 2) induction of tolerance to immunogenic proteins that are given to patients (hemophilia, as an example); 3) rituximab has therapeutic efficacy in arthritis and other autoimmune disorders; CART19 may work as well or better.

In some instances, the CART19 cells can be used to eliminate all B cell subsets (e.g,. naïve, memory, plasma cell precursors, and "suppressive B regs"). Bregs may contribute to the immunosuppression of some cancers and therefore CART19 might improve immune responses by removing the Bregs.

## Claims

1. A cell genetically modified to express an anti-CD19 CAR wherein the CAR comprises a CD19 antigen binding domain, a costimulatory signaling region, and a CD3 zeta signaling domain, for use in a method for preventing or delaying rejection of a transplanted tissue or organ in a subject, the method comprising administering to the subject an effective amount of said genetically modified cell before, at the same time as, or after the administration of the transplanted tissue or organ, wherein the CD19 antigen binding domain targets the CD19 B cell surface marker, wherein the genetically modified cell depletes B cells, and wherein B cell aplasia is sustained for eighteen months after the administration of the genetically modified cell to the subject, wherein the tissue or organ is heart, heart valve, lung, kidney, liver, pancreas, intestine, skin, blood vessel, bone marrow, stem cell, bone or islet cell.

2. A cell genetically modified to express an anti-CD19 CAR, wherein the CAR comprises a CD19 antigen binding domain, a costimulatory signaling region, and a CD3 zeta signaling domain, for use in a method for treating graft versus host disease (GVHD) in a subject having received a tissue or organ transplant, the method comprising administering to the subject an effective amount of said genetically modified cell before, at the same time as, or after the administration of the transplanted tissue or organ, wherein the antigen binding domain targets a CD19 B cell surface marker, wherein the genetically modified cell depletes B cells, thereby treating GVHD in the subject, and wherein B cell aplasia is sustained for eighteen months after the administration of the genetically modified cell to the subject, wherein the tissue or organ is heart, heart valve, lung, kidney, liver, pancreas, intestine, skin, blood vessel, bone marrow, stem cell, bone or islet cell.

3. The genetically modified cell for use as in claim 2, wherein the genetically modified cell is administered at the same time as a transplanted tissue or organ.

4. The genetically modified cell for use as in claim 3, wherein the genetically modified cell is administered before the administration of the transplanted tissue or organ.

5. The genetically modified cell for use as in claim 3, wherein the genetically modified cell is administered after the administration of the transplanted tissue or organ.

6. The genetically modified cell for use as in claim 1, wherein the method for preventing or delaying rejection of a transplanted tissue or organ in a subject further comprises administration of a genetically modified cell to said tissue or organ before said tissue or organ is transplanted into the subject.

7. The genetically modified cell for use as in claim 1, wherein the method for preventing or delaying rejection of a transplanted tissue or organ in a subject further comprises evaluation of the presence of CAR expressing cells in the subject at least once at twelve or eighteen months after the administration of the cell genetically modified to express an anti CD19 CAR.

8. The genetically modified cell for use as in claim 1, wherein the method for preventing or delaying rejection of a transplanted tissue or organ in a subject further comprises the evaluation of the depletion of B cells in the subject at least once at twelve or eighteen months after the administration of the cell genetically modified to express an anti-CD19 CAR.

9. The genetically modified cell for use as in claim 2, wherein the method for treating graft versus host disease (GVHD) further comprises administration of said genetically modified cell to a tissue or organ before said tissue or organ is transplanted into the subject.

10. The genetically modified cell for use as in claim 2, wherein the method for treating graft versus host disease (GVHD) further comprises the evaluation of the presence of CAR expressing cells in the subject at least once at twelve or eighteen months after the administration of the cell genetically modified to express an anti-CD19 CAR.

11. The genetically modified cell for use as in claim 2, wherein the method for treating graft versus host disease (GVHD) further comprises the evaluation of the depletion of B cells in the subject at least once at twelve or eighteen months after the administration of the cell genetically modified to express an anti-CD19 CAR.

## Patentansprüche

1. Zelle, gentechnisch verändert zur Expression eines Anti-CD19-CAR, wobei der CAR eine CD19-Antigenbindungsdomäne, eine Costimulator-Signalgebungsregion und eine CD3-zeta-Signalgebungsdomäne umfasst, zur Verwendung bei einem Verfahren zur Vorbeugung oder Verzögerung der Abstoßung eines transplantierten Gewebes oder Organs bei einem Individuum, wobei das Verfahren Verabreichen einer wirksamen Menge der gentechnisch veränderten Zelle an das Individuum vor, gleichzeitig mit oder nach der Verabreichung des transplantierten Gewebes oder Organs umfasst, wobei die CD19-Antigenbindungsdomäne auf den CD19-B-Zelloberflächenmarker zielt, wobei die gentechnisch veränderte Zelle die Depletion von B-Zellen bewirkt und wobei B-Zell-Aplasie nach der Verabreichung der gentechnisch veränderten Zelle an das Individuum achtzehn Monate anhält, wobei es sich bei dem Gewebe bzw. Organ um Herz, Herzklappe, Lunge, Niere, Leber, Pankreas, Darm, Haut, Blutgefäß, Knochenmark, Stammzelle, Knochen oder Inselzelle handelt.

2. Zelle, gentechnisch verändert zur Expression eines Anti-CD19-CAR, wobei der CAR eine CD19-Antigenbindungsdomäne, eine Costimulator-Signalgebungsregion und eine CD3-zeta-Signalgebungsdomäne umfasst, zur Verwendung bei einem Verfahren zur Behandlung von Graft-versus-Host-Krankheit (GVHD) bei einem Individuum, das ein Gewebe- oder Organtransplantat erhalten hat, wobei das Verfahren Verabreichen einer wirksamen Menge der gentechnisch veränderten Zelle an das Individuum vor, gleichzeitig mit oder nach der Verabreichung des transplantierten Gewebes oder Organs umfasst, wobei die Antigenbindungsdomäne auf einen CD19-B-Zelloberflächenmarker zielt, wobei die gentechnisch veränderte Zelle die Depletion von B-Zellen bewirkt, wodurch GVHD bei dem Individuum behandelt wird, und wobei B-Zell-Aplasie nach der Verabreichung der gentechnisch veränderten Zelle an das Individuum achtzehn Monate anhält, wobei es sich bei dem Gewebe bzw. Organ um Herz, Herzklappe, Lunge, Niere, Leber, Pankreas, Darm, Haut, Blutgefäß, Knochenmark, Stammzelle, Knochen oder Inselzelle handelt.

3. Gentechnisch veränderte Zelle zur Verwendung gemäß Anspruch 2, wobei die gentechnisch veränderte Zelle zur gleichen Zeit wie ein transplantiertes Gewebe oder Organ verabreicht wird.

4. Gentechnisch veränderte Zelle zur Verwendung gemäß Anspruch 3, wobei die gentechnisch veränderte Zelle vor der Verabreichung des transplantierten Gewebes oder Organs verabreicht wird.

5. Gentechnisch veränderte Zelle zur Verwendung gemäß Anspruch 3, wobei die gentechnisch veränderte Zelle nach der Verabreichung des transplantierten Gewebes oder Organs verabreicht wird.

6. Gentechnisch veränderte Zelle zur Verwendung gemäß Anspruch 1, wobei das Verfahren zur Vorbeugung oder Verzögerung der Abstoßung eines transplantierten Gewebes oder Organs bei einem Individuum ferner Verabreichung einer gentechnisch veränderten Zelle an das Gewebe oder Organ vor dem Transplantieren des Gewebes oder Organs in das Individuum umfasst.

7. Gentechnisch veränderte Zelle zur Verwendung gemäß Anspruch 1, wobei das Verfahren zur Vorbeugung oder Verzögerung der Abstoßung eines transplantierten Gewebes oder Organs bei einem Individuum ferner wenigstens einmal eine Bewertung des Vorliegens CAR exprimierender Zellen beim Individuum zwölf oder achtzehn Monate nach der Verabreichung der zur Expression eines Anti-CD19-CAR gentechnisch veränderten Zelle umfasst.

8. Gentechnisch veränderte Zelle zur Verwendung gemäß Anspruch 1, wobei das Verfahren zur Vorbeugung oder Verzögerung der Abstoßung eines transplantierten Gewebes oder Organs bei einem Individuum ferner wenigstens einmal die Bewertung der Depletion von B-Zellen beim Individuum zwölf oder achtzehn Monate nach der Verabreichung der zur Expression eines Anti-CD19-CAR gentechnisch veränderten Zelle umfasst.

9. Gentechnisch veränderte Zelle zur Verwendung gemäß Anspruch 2, wobei das Verfahren zur Behandlung von Graft-versus-Host-Krankheit (GVHD) ferner Verabreichung der gentechnisch veränderten Zelle an ein Gewebe oder Organ vor dem Transplantieren des Gewebes oder Organs in das Individuum umfasst.

10. Gentechnisch veränderte Zelle zur Verwendung gemäß Anspruch 2, wobei das Verfahren zur Behandlung von Graft-versus-Host-Krankheit (GVHD) ferner wenigstens einmal die Bewertung des Vorliegens CAR exprimierender Zellen beim Individuum zwölf oder achtzehn Monate nach der Verabreichung der zur Expression eines Anti-CD19-CAR gentechnisch veränderten Zelle umfasst.

11. Gentechnisch veränderte Zelle zur Verwendung gemäß Anspruch 2, wobei das Verfahren zur Behandlung von Graft-versus-Host-Krankheit (GVHD) ferner wenigstens einmal die Bewertung der Depletion von B-Zellen beim Individuum zwölf oder achtzehn Monate nach der Verabreichung der zur Expression eines Anti-CD19-CAR gentechnisch veränderten Zelle umfasst.

## Revendications

1. Cellule génétiquement modifiée pour exprimer un CAR anti-CD19, dans laquelle le CAR comprend un domaine de liaison à l'antigène CD19, une région de signalisation co-stimulatrice et un domaine de signalisation de CD3 zêta, destinée à être utilisée dans un procédé destiné à prévenir ou à retarder un rejet de tissu ou d'organe greffé chez un sujet, le procédé comprenant une administration au sujet d'une quantité efficace de ladite cellule génétiquement modifiée avant, en même temps que ou après l'administration du tissu ou de l'organe greffé, dans laquelle le domaine de liaison à l'antigène CD19 cible le marqueur de surface CD19 des lymphocytes B, la cellule génétiquement modifiée épuisant les lymphocytes B, et dans laquelle l'aplasie des lymphocytes B est prolongée pendant dix-huit mois après l'administration au sujet de la cellule génétiquement modifiée, dans laquelle le tissu ou l'organe est le cœur, une valve cardiaque, un poumon, un rein, le foie, le pancréas, un intestin, la peau, un vaisseau sanguin, la moelle osseuse, une cellule souche, une cellule d'os ou d'îlots.

2. Cellule génétiquement modifiée pour exprimer un CAR anti-CD19, dans laquelle le CAR comprend un domaine de liaison à l'antigène CD19, une région de signalisation co-stimulatrice et un domaine de signalisation de CD3 zêta, destinée à être utilisée dans un procédé destiné à traiter la maladie du greffon contre l'hôte (GVHD) chez un sujet ayant reçu une greffe de tissu ou d'organe, le procédé comprenant une administration au sujet d'une quantité efficace de ladite cellule génétiquement modifiée avant, en même temps que ou après l'administration du tissu ou de l'organe greffé, dans laquelle le domaine de liaison à l'antigène cible un marqueur de surface CD19 des lymphocytes B, la cellule génétiquement modifiée épuisant les lymphocytes B, en traitant de ce fait la GVHD chez le sujet, et dans laquelle l'aplasie des lymphocytes B est prolongée pendant dix-huit mois après l'administration au sujet de la cellule génétiquement modifiée, dans laquelle le tissu ou l'organe est le cœur, une valve cardiaque, un poumon, un rein, le foie, le pancréas, un intestin, la peau, un vaisseau sanguin, la moelle osseuse, une cellule souche, une cellule d'os ou d'îlots.

3. Cellule génétiquement modifiée destinée à être utilisée comme dans la revendication 2, la cellule génétiquement modifiée étant administrée en même temps qu'un tissu ou qu'un organe greffé.

4. Cellule génétiquement modifiée destinée à être utilisée comme dans la revendication 3, la cellule génétiquement modifiée étant administrée avant l'administration du tissu ou de l'organe greffé.

5. Cellule génétiquement modifiée destinée à être utilisée comme dans la revendication 3, la cellule génétiquement modifiée étant administrée après l'administration du tissu ou de l'organe greffé.

6. Cellule génétiquement modifiée destinée à être utilisée comme dans la revendication 1, dans laquelle le procédé destiné à prévenir ou à retarder un rejet de tissu ou d'organe greffé chez un sujet comprend en outre une administration d'une cellule génétiquement modifiée au dit tissu ou au dit organe avant que ledit tissu ou ledit organe soit transplanté dans le sujet.

7. Cellule génétiquement modifiée destinée à être utilisée comme dans la revendication 1, dans laquelle le procédé destiné à prévenir ou à retarder un rejet de tissu ou d'organe greffé chez un sujet comprend en outre une évaluation de la présence de cellules exprimant CAR chez le sujet, au moins une fois, douze ou dix-huit mois après l'administration de la cellule génétiquement modifiée pour exprimer un CAR anti-CD19.

8. Cellule génétiquement modifiée destinée à être utilisée comme dans la revendication 1, dans laquelle le procédé destiné à prévenir ou à retarder un rejet de tissu ou d'organe greffé chez un sujet comprend en outre l'évaluation de l'épuisement des lymphocytes B chez le sujet, au moins une fois, douze ou dix-huit mois après l'administration de la cellule génétiquement modifiée pour exprimer un CAR anti-CD19.

9. Cellule génétiquement modifiée destinée à être utilisée comme dans la revendication 2, dans laquelle le procédé destiné à traiter la maladie du greffon contre l'hôte (GVHD) comprend en outre une administration de ladite cellule génétiquement modifiée à un tissu ou à un organe avant que ledit tissu ou ledit organe soit transplanté dans le sujet.

10. Cellule génétiquement modifiée destinée à être utilisée comme dans la revendication 2, dans laquelle le procédé destiné à traiter la maladie du greffon contre l'hôte (GVHD) comprend en outre l'évaluation de la présence de cellules exprimant CAR chez le sujet, au moins une fois, douze ou dix-huit mois après l'administration de la cellule génétiquement modifiée pour exprimer un CAR anti-CD19.

11. Cellule génétiquement modifiée destinée à être utilisée comme dans la revendication 2, dans laquelle le procédé destiné à traiter la maladie du greffon contre l'hôte (GVHD) comprend en outre l'évaluation de l'épuisement des lymphocytes B chez le sujet, au moins une fois, douze ou dix-huit mois après l'administration de la cellule génétiquement modifiée pour exprimer un CAR anti-CD19.
